# EUROPEAN PATENT APPLICATION

(11) **EP 4 596 986 A1**
(43) Date of publication of application: **06.08.2025**
(21) Application number: 24741720.7
(22) Date of filing: 11.01.2024
(51) Int. Cl.: F24F 8/22, F24F 8/108, F24F 8/80, F24F 13/20, F24F 11/65, F24F 110/64

(54) **AIR CLEANING DEVICE AND CONTROL METHOD THEREOF**

(30) Priority: 13.01.2023 KR 20230005525
(71) Applicant: Samsung Electronics Co., Ltd., Suwon-si, Gyeonggi-do 16677 (KR)
(72) Inventor: CHU, Euysung, Suwon-si, Gyeonggi-do 16677 (KR); LEE, Taeyong, Suwon-si, Gyeonggi-do 16677 (KR); LIM, Youngseok, Suwon-si, Gyeonggi-do 16677 (KR)
(74) Representative: Walaski, Jan Filip
(86) International application number: PCT/KR2024/000533
(87) International publication number: WO 2024/151099

(57) **Abstract**

An air cleaning device including a cleaning unit including a first housing including a first inlet and first outlet, and a first passage from the first inlet to the first outlet, a first fan to generate an air flow through the first passage, and a dust collection filter in the first passage to collect dust from the air flow moving through the first passage; and a sterilizing unit including a second housing including a second inlet connected to the first outlet, a second outlet, and a second passage extending from the second inlet to the second outlet, a second fan to generate an air flow through the second passage, and an ultraviolet light source to irradiate ultraviolet rays to the air flow moving through the second passage. The air cleaning device is operable in a simultaneous mode, two different individual modes, and a standby mode.

## Description

### Technical Field

Embodiments provide an air cleaning device and a control method of the air cleaning device.

### Background Art

Air cleaning devices are used to remove contaminants from the air. Air cleaning devices may remove chemicals or the like that cause bad odors, such as bacteria, viruses, fungi, and fine dust, present in suctioned air.

Air cleaning devices may include an inlet to suck in contaminated air, an outlet to discharge purified air, and a blowing fan that causes air flow.

Air cleaning devices may include a filter to purify contaminated indoor air. Air sucked into air cleaning devices may pass through a filter to remove contaminants and purify the air into clean air, and the purified air may be discharged to the outside of air cleaning devices.

In air cleaning devices, an air flow passage through which air flows from an inlet to an outlet may be formed. The air flow passage may be provided in various ways according to operation purposes of air cleaning devices. Shapes and positions of the inlet and the outlet may be determined in various ways in consideration of a shape of the air flow passage.

### Disclosure

### Technical Problem

According to an embodiment of the disclosure, an air cleaning device may include a cleaning unit and a sterilizing unit connected to the cleaning unit.

The cleaning unit may include a first housing including a first inlet and a first outlet, and a first passage extending from the first inlet to the first outlet, a first fan configured to generate an air flow through the first passage, and a dust collection filter in the first passage to collect dust from the air flow generated by the first fan and moving through the first passage, so that the cleaning unit is operable to generate, and collect the dust from, the air flow moving through the first passage.

The sterilizing unit may include a second housing including a second inlet connected to the first outlet, a second outlet, and a second passage extending from the second inlet to the second outlet, a second fan configured to generate an air flow through the second passage, and an ultraviolet light source to irradiate ultraviolet rays to the air flow generated by the second fan and moving through the second passage, so that the sterilizing unit is operable to generate, and irradiate the ultraviolet rays to, the air flow moving through the second passage.

The air cleaning device may be configured to be selectively operable in a simultaneous mode in which both the cleaning unit and the sterilizing unit are operated, first and second individual modes in which one of the cleaning unit and the sterilizing unit is operated alone, and a standby mode in which neither the cleaning unit nor the sterilizing unit are operated.

In the simultaneous mode, the first fan and the second fan each generate air flows so that air is sucked in through the first inlet, the air sucked in through the first inlet moves along the first passage and is purified by the dust collection filter, a first portion of the purified air passes through the first outlet and into the second inlet, a second portion of the purified air is discharged to an outside through the first outlet, the first portion of the purified air passed into the second inlet moves along the second passage and is sterilized by the ultraviolet light source, and the sterilized air is discharged to the outside through the second outlet.

According to an embodiment of the disclosure, a method of controlling an air cleaning device may include measuring at least one of a concentration of dust or a concentration of floating bacteria in surrounding air of the air cleaning device.

According to an embodiment of the disclosure, the method may include, based on results of the measuring, controlling the air cleaning device to operate in one operation mode among a simultaneous mode in which both the cleaning unit and the sterilizing unit are operated, first and second individual modes in which one of the cleaning unit and the sterilizing unit is operated alone, and a standby mode in which neither the cleaning unit nor the sterilizing unit are operated.
In the simultaneous mode the first fan and the second fan each generate air flows so that air is sucked in through the first inlet, the air sucked in through the first inlet moves along the first passage and is purified by the dust collection filter, a first portion of the purified air passes through the first outlet and into the second inlet, a second portion of the purified air is discharged to an outside through the first outlet, the first portion of the purified air passed into the second inlet moves along the second passage and is sterilized by the ultraviolet light source, and the sterilized air is discharged to the outside through the second outlet.

### Description of Drawings

FIG. 1 is a perspective view of an air cleaning device according to an embodiment of the disclosure.
FIG. 2 is an exploded perspective view of some components of an air cleaning device according to an embodiment of the disclosure.
FIG. 3 is an exploded view of some components of a cleaning unit according to an embodiment of the disclosure.
FIG. 4 is an exploded view of some components of a cleaning unit according to an embodiment of the disclosure.
FIG. 5 is a side cross-sectional view of a cleaning unit according to an embodiment of the disclosure.
FIG. 6 is an exploded view of some components of a sterilizing unit of an air cleaning device according to an embodiment of the disclosure.
FIG. 7 is an exploded view of some components of a sterilizing unit of an air cleaning device according to an embodiment of the disclosure.
FIG. 8 is a diagram for explaining air flowing in a sterilizing unit of an air cleaning device according to an embodiment of the disclosure.
FIG. 9 is a side cross-sectional view of a sterilizing unit of an air cleaning device according to an embodiment of the disclosure.
FIG. 10 is a diagram illustrating an intermediate duct of an air cleaning device according to an embodiment of the disclosure and some peripheral components thereof.
FIG. 11 is an enlarged cross-sectional view of some components of an air cleaning device according to an embodiment of the disclosure.
FIG. 12 is a block diagram illustrating a configuration of an air cleaning device according to an embodiment of the disclosure.
FIG. 13 is a cross-sectional view for explaining an operation of an air cleaning device according to an embodiment of the disclosure in a simultaneous mode.
FIG. 14 is a cross-sectional view for explaining an operation of an air cleaning device according to an embodiment of the disclosure in a first individual mode.
FIG. 15 is a cross-sectional view for explaining an operation of an air cleaning device according to an embodiment of the disclosure in a second individual mode.
FIG. 16 is a cross-sectional view for explaining an operation of an air cleaning device according to an embodiment of the disclosure in a standby mode.
FIG. 17 is a flowchart illustrating a control method of an air cleaning device according to an embodiment of the disclosure.
FIG. 18 is a flowchart illustrating an operation of a cleaning unit according to an embodiment of the disclosure.
FIG. 19 is a flowchart illustrating an operation of a sterilizing unit according to an embodiment of the disclosure.
FIG. 20 is a diagram for explaining an operation of a cleaning unit according to a concentration of dust in surrounding air in an air cleaning device according to an embodiment of the disclosure.
FIG. 21 is a diagram for explaining an operation of a sterilizing unit according to a concentration of floating bacteria in surrounding air in an air cleaning device according to an embodiment of the disclosure.
FIG. 22 is a flowchart illustrating a control method of an air cleaning device according to an embodiment of the disclosure.
FIG. 23 is a flowchart for explaining an operation of an air cleaning device according to an embodiment of the disclosure in a simultaneous mode.
FIG. 24 is a diagram illustrating an operation mode, determined by a user input, of an air cleaning device according to an embodiment of the disclosure.

### Mode for Invention

Embodiments of the disclosure will be described more fully with reference to the accompanying drawings. In the drawings, like reference numerals or symbols refer to like components or elements performing substantially the same functions.

It will be understood that, although the terms including an ordinal number such as "first", "second", etc., may be used herein to describe various elements or components, these elements or components should not be limited by the terms. The terms are only used to distinguish one element or component from another element or component. For example, as used herein, a first element or component may be termed a second element or component without departing from the scope of the disclosure, and similarly, a second element or component may be termed a first element or component. As used herein, the term "and/or" includes any and all combinations of one or more of the associated listed items.

The terms used herein are for the purpose of describing an embodiment of the disclosure and is not intended to limit the disclosure. As used herein, singular forms are intended to include the plural forms as well, unless the context clearly indicates otherwise. It will be further understood that the terms "comprises" and/or "includes" when used in this specification, specify the presence of stated features, numbers, steps, operations, elements, components, or combinations thereof, but do not preclude the presence or addition of one or more other features, numbers, steps, operations, elements, components, or combinations thereof. In the drawings, like reference numerals represent like elements performing substantially the same functions.

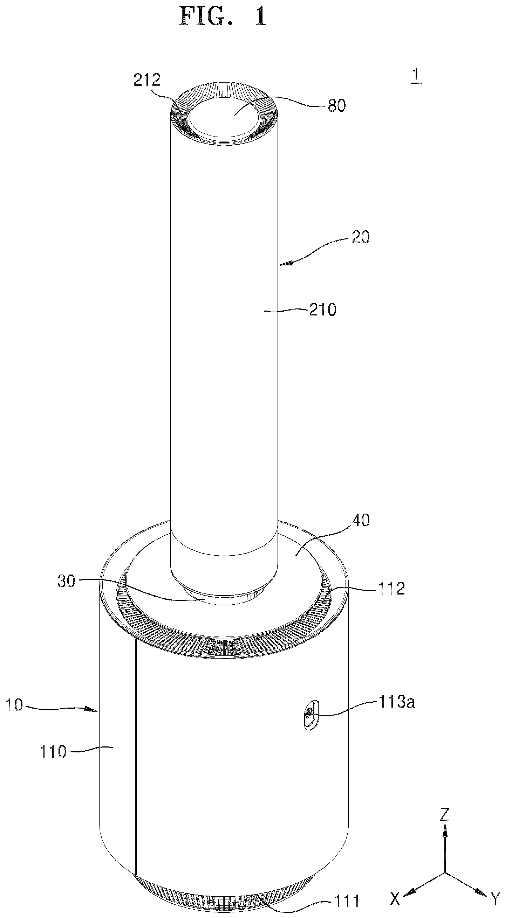

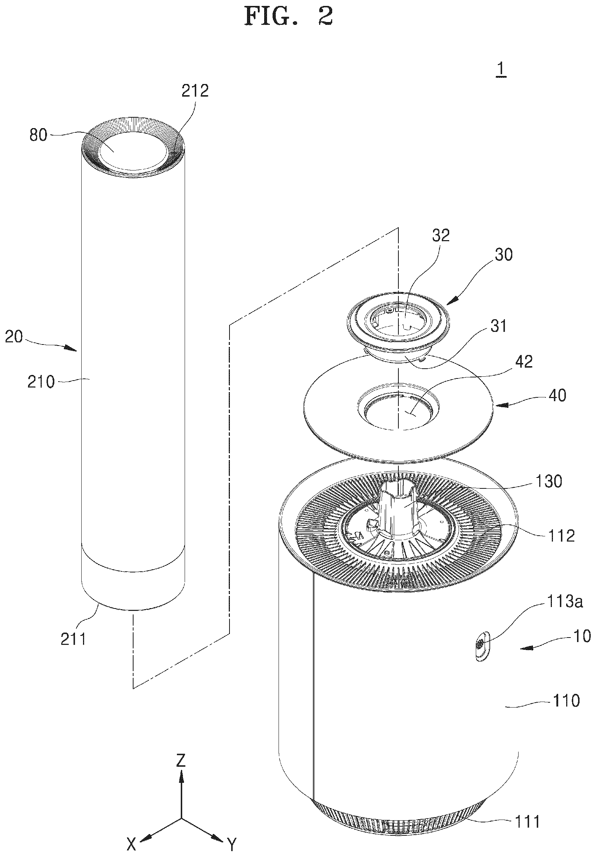

FIG. 1 is a perspective view of an air cleaning device 1 according to an embodiment of the disclosure. FIG. 2 is an exploded perspective view of some components of the air cleaning device 1 according to an embodiment of the disclosure.

Referring to FIGS. 1 and 2, the air cleaning device 1 according to an embodiment of the disclosure may be provided to control air properties such as temperature, humidity, cleaning state, and air flow. For example, the air cleaning device 1 may improve the purity of indoor air. The air cleaning device 1 according to an embodiment of the disclosure may include a cleaning unit 10 and a sterilizing unit 20 connected to the cleaning unit 10.

The cleaning unit 10 may purify air introduced from the outside and discharge purified air back to the outside. The air may be introduced into the cleaning unit 10 through a first inlet 111, and the purified air may be discharged through a first outlet 112.

The sterilizing unit 20 may sterilize the introduced air and discharge the sterilized air to the outside. The air may be introduced into the sterilizing unit 20 through a second inlet 211, and the sterilized air may be discharged through a second outlet 212.

The sterilizing unit 20 may be disposed in an upper portion of the cleaning unit 10 so as to be supported on the cleaning unit 10. In another expression, the cleaning unit 10 may be disposed in a lower portion of the sterilizing unit 20 to support the sterilizing unit 20. The sterilizing unit 20 may be separately assembled with the cleaning unit 10.

The sterilizing unit 20 may be connected to the cleaning unit 10. As an example, the air cleaning device 1 may include an intermediate duct 30 and a discharge passage guide 40 disposed between the sterilizing unit 20 and the cleaning unit 10.

The intermediate duct 30 may be provided between a first housing 110 of the cleaning unit 10 and a second housing 210 of the sterilizing unit 20, and connect the first housing 110 to the second housing 210. The discharge passage guide 40 may guide the flow of air discharged through the first outlet 112 of the cleaning unit 10. This will be described below.

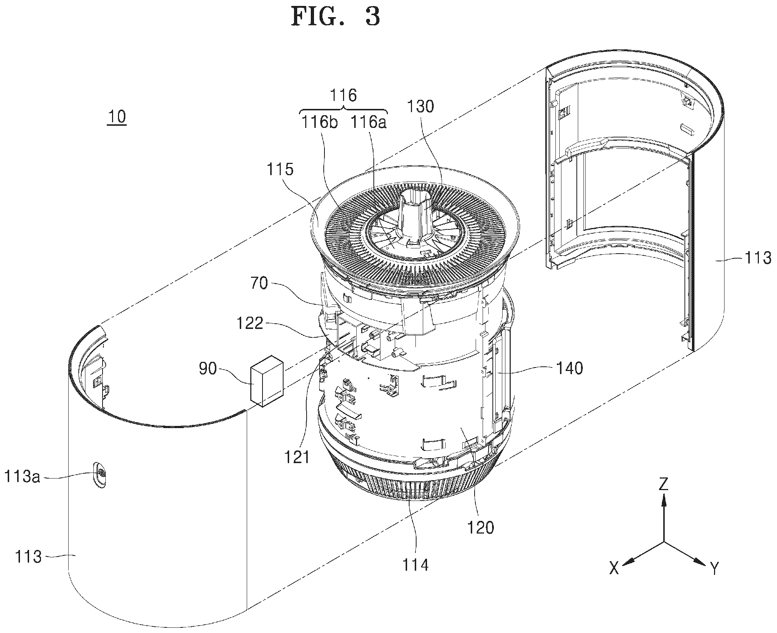

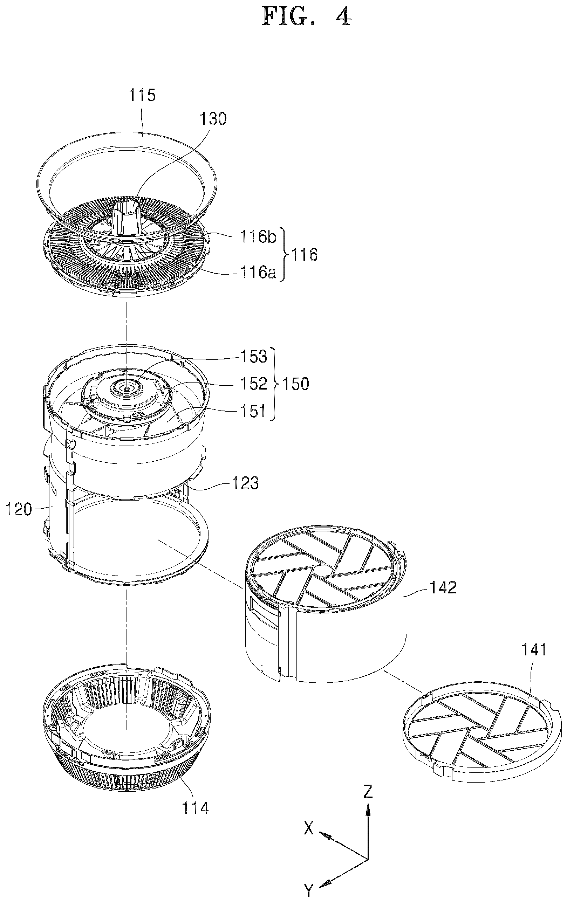

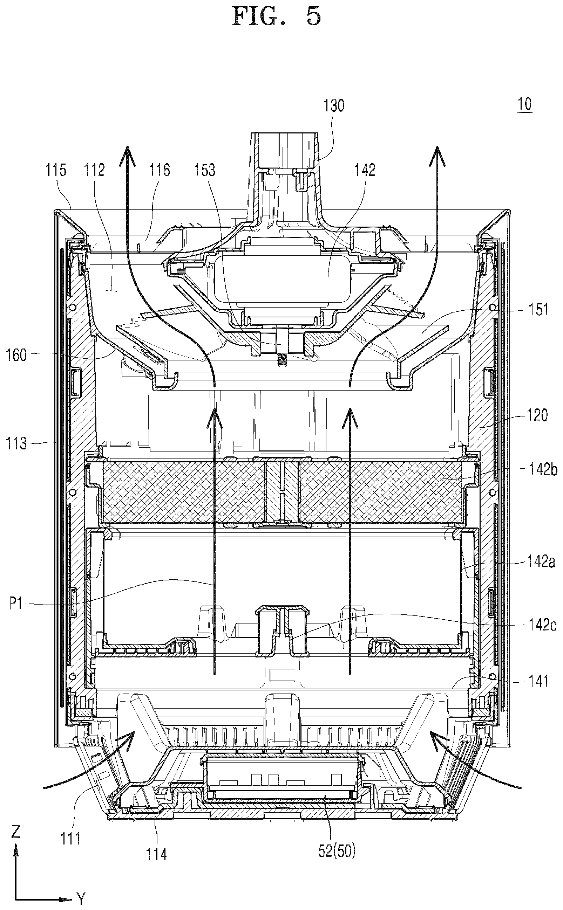

FIG. 3 is an exploded view of some components of the cleaning unit 10 according to an embodiment of the disclosure. FIG. 4 is an exploded view of some components of the cleaning unit 10 according to an embodiment of the disclosure. FIG. 5 is a side cross-sectional view of the cleaning unit 10 according to an embodiment of the disclosure.

Referring to FIGS. 1 and 3 to 5, the cleaning unit 10 according to an embodiment of the disclosure may include the first housing 110 forming the exterior thereof, and a first internal frame 120 provided to support various components inside the first housing 110.

The first housing 110 in which a first passage P1 is provided may be configured to accommodate various components of the cleaning unit 10.

The first housing 110 may include the first inlet 111 and the first outlet 112. The first inlet 111 may be provided so that external air of the first housing 110 is introduced into the first housing 110. The first outlet 112 may be provided so that internal air of the first housing 110 is discharged to the outside of the first housing 110. The first passage P1 extending from the first inlet 111 to the first outlet 112 may be provided inside the first housing 110.

For example, the first inlet 111 may be formed in a lower portion of the first housing 110. The first outlet 112 may be formed in an upper portion of the first housing 110. At least a part of the first passage P1 may extend in a vertical direction, for example, in a Z direction. Accordingly, the air introduced into the first housing 110 through the first inlet 111 may move upward along the first passage P1. However, the arrangement of the first inlet 111 and the first outlet 112 is not limited thereto, and the first inlet 111 and the first outlet 112 may be formed at various positions.

The first housing 110 may include a housing body 113, a base 114, and a cover or grill assembly 116. The housing body 113, the base 114, and the grill assembly 116 may each form a part of the exterior of the cleaning unit 10.

The housing body 113 may form side surfaces of the cleaning unit 10 in a horizontal direction. The housing body 113 may be provided to cover various components of the cleaning unit 10 in the horizontal direction. The housing body 113 may connect the base 114 to the grill assembly 116.

The housing body 113 may extend in a direction parallel to the first passage P1. For example, the housing body 113 may extend longitudinally in the vertical direction.

The housing body 113 may have an approximately cylindrical shape with a space therein. However, the shape of the housing body 113 is not limited thereto, and the housing body 113 may have various shapes in accordance with the external shape of the cleaning unit 10.

The housing body 113 may be divided into a plurality of panels. The plurality of panels may be disassembled and assembled. The housing body 113 may be easily disassembled or assembled, and user accessibility to components arranged inside the housing body 113 may be improved. However, the structure of the housing body 113 is not limited thereto, and the housing body 113 may be integrally formed into a single configuration.

The base 114 may be provided at a lower portion of the housing body 113. The base 114 may form a bottom surface of the cleaning unit 10. When the cleaning unit 10 is placed on the ground, the base 114 may support the cleaning unit 10. The base 114 may be coupled to a lower portion of the first internal frame 120.

Side surfaces of the base 114 may be formed in a circumferential direction of the base 114. The side surfaces of the base 114 may extend in the vertical direction or in a direction at a certain angle with the vertical direction.

The first inlet 111 may be formed in the base 114. The first inlet 111 may be formed in the side surfaces of the base 114. The first inlet 111 may be disposed along the circumference of the base 114. The side surfaces of the base 114 may have a grill shape to facilitate the entry and exit of air.

The grill assembly 116 may be provided on an upper portion of the housing body 113. The grill assembly 116 may be disposed on an upper portion of a first fan 150. The grill assembly 116 may form an upper surface of the cleaning unit 10. The grill assembly 116 may be formed to cover an interior space of the first housing 110 from above.

The grill assembly 116 may have a grill shape so that air may enter and exit through the first outlet 112. The grill shape may be disposed along the circumference of an upper surface of the first housing 110. However, the arrangement of the grill shape is not limited thereto and may vary.

The first internal frame 120 may be disposed inside the first housing 110. The first internal frame 120 may be configured to support the grill assembly 116. The first internal frame 120 may be coupled to an inner surface of the first housing 110. The first internal frame 120 may support an inner surface of the housing body 113.

The first internal frame 120 may be provided to support various components of the cleaning unit 10. Components of the cleaning unit 10, for example, a dust collection filter 140 and the first fan 150, may be supported by the first internal frame 120.

The first internal frame 120 may be covered by the first housing 110. As an example, the first inner frame 120 in a lateral direction thereof may be covered by the housing body 113 in the horizontal direction. The first internal frame 120 in a downward direction thereof may be covered by the base 114 in the vertical direction. The first internal frame 120 in an upward direction thereof may be covered by the grill assembly 116 in the vertical direction.

The first internal frame 120 may be divided into a plurality of frames. The plurality of frames constituting the first internal frame 120 may form the integrated first internal frame 120 while coupled to each other. Accordingly, the first internal frame 120 may be easily disassembled or assembled, and user accessibility to the internal configuration of the first internal frame 120 may be improved. However, the first internal frame 120 is not limited thereto, and may be integrally formed into a single configuration.

A substantially cylindrical space may be formed inside the first internal frame 120. The dust collection filter 140, the first fan 150, etc., may be disposed in an internal space of the first internal frame 120. The first passage P1 may be provided in the internal space of the first internal frame 120. The first internal frame 120 may cover an outer side of the first passage P1.

However, the disclosure is not limited thereto, and the first internal frame 120 may be formed to have various shapes. As an example, the space formed inside the first internal frame 120 may have a shape other than a cylinder shape. Alternatively, as an example, the first internal frame 120 may be provided to support various components of the cleaning unit 10 without forming a space therein or covering the outer side of the first passage P1.

The first internal frame 120 described above is only an example of a configuration provided to support various components of the cleaning unit 10, and the disclosure is not limited thereto.

The cleaning unit 10 according to an embodiment of the disclosure may include the first fan 150 disposed inside the first housing 110. The first fan 150 may be disposed on the first passage P1. The first fan 150 may be disposed between the first inlet 111 and the first outlet 112. The first fan 150 may generate flow. As the first fan 150 rotates, flow may be generated in a direction from the first inlet 111 to the first outlet 112. Accordingly, external air of the first housing 110 may be introduced through the first inlet 111, and the introduced air may move along the first passage P1 and may be discharged through the first outlet 112.

The first fan 150 may be configured as an axial flow type. The first fan 150 may include a blade 151, a motor 152 that supplies power to the blade 151, and a fan rotation shaft 153 that is connected to the blade 151 and the motor 152 and transmits power generated from the motor 152 to the blade 151. However, the type and configuration of the first fan 150 are not limited thereto and may be modified as needed. For example, the first fan 150 may be configured as a centrifugal type.

The first fan 150 may be disposed between the dust collection filter 140 and the first outlet 112. When the first fan 150 is driven, air introduced into the first inlet 111 may sequentially pass through the dust collection filter 140 and the first fan 150 and be discharged through the first outlet 112. However, the arrangement of the first fan 150 is not limited thereto and may vary. For example, unlike the drawing, the first fan 150 may be disposed between the first inlet 111 and the dust collection filter 140.

The dust collection filter 140 may remove or collect foreign substances in the air introduced into the first housing 110. The dust collection filter 140 may be provided in the first passage P1. When the first fan 150 is driven, the air introduced through the first inlet 111 may move along the first passage P1 and pass through the dust collection filter 140. Foreign substances in the air moving along the first passage P1 may be removed by the dust collection filter 140. The air from which foreign substances have been removed by the dust collection filter 140 may be discharged through the first outlet 112.

The dust collection filter 140 may have a shape corresponding to the shape of the first housing 110. For example, the dust collection filter 140 may have a cylindrical structure. However, the shape of the dust collection filter 140 is not limited thereto and may vary in accordance with the shape of the first housing 110.

The dust collection filter 140 may be detachably mounted on the first internal frame 120. As an example, the first internal frame 120 may include a dust collection filter mounting opening 123 formed to allow the dust collection filter 140 to be inserted into or withdrawn from the inside of the first internal frame 120. The interior of the first internal frame 120 may be opened through the dust collection filter mounting opening 123.

As an example, the dust collection filter mounting opening 123 may be formed in one side of the first internal frame 120. The dust collection filter 140 may be inserted into and mounted on one side of the first internal frame 120, or may be withdrawn and separated from one side of the first internal frame 120.

With this configuration, a user may easily separate or assemble the dust collection filter 140 from or with the first internal frame 120, and may easily repair, replace, or clean the dust collection filter 140.

However, the disclosure is not limited thereto, and the dust collection filter 140 may be mounted on the first internal frame 120 in various ways. Alternatively, the dust collection filter 140 may be supported by various configurations so as to be disposed on the first passage P1.

The dust collection filter 140 may include an electric dust collection filter 142 and a pre-filter 141.

The pre-filter 141 may be provided in the first passage P1. The pre-filter 141 may be disposed on a passage facing the electric dust collection filter 142. The pre-filter 141 may be disposed between the first inlet 111 and the electric dust collection filter 142 in the first passage P1.

The pre-filter 141 may be provided to primarily collect foreign substances in the air sucked into the first inlet 111. The pre-filter 141 may be provided to separate relatively large foreign substances from the air. For example, the pre-filter 141 may be configured to filter out foreign substances of a first size.

The electric dust collection filter 142 may be provided on the first passage P1. The electric dust collection filter 142 may be disposed downstream of the first passage P1 rather than the pre-filter 141. The electric dust collection filter 142 may be provided to separate foreign substances smaller than those separated by the pre-filter 141. For example, the electric dust collection filter 142 may be configured to filter out foreign substances of a second size smaller than the first size.

The electric dust collection filter 142 may be configured to collect dust by using electrostatic force. The electric dust collection filter 142 may receive power from a power supplier 70, which will be described below. When power is applied to the electric dust collection filter 142, an electric field may be formed in the electric dust collection filter 142. Foreign substances such as dust in the air may be collected due to the electric field formed in the electric dust collection filter 142. In addition, floating bacteria such as bacteria and viruses may be decomposed due to the electric field formed in the electric dust collection filter 142. That is, the electric dust collection filter 142 may perform dust collection and sterilization functions.

The electric dust collection filter 142 may include a filter case 142a forming the exterior of the electric dust collection filter 142, a charger 142c, and a dust collector 142b.

The filter case 142a may support the dust collector 142b and the charger 142c. The filter case 142a may be supported on the first internal frame 120. At least one electrode receiving power from the power supplier 70 may be provided in the filter case 142a. As an example, an electrode of the first internal frame 120 that is electrically connected to the power supplier 70 may be provided in the first internal frame 120, and may be connected to an electrode of the filter case 142a when the dust collection filter 140 is mounted in the first internal frame 120. The electrode of the first internal frame 120 or the electrode of the filter case 142a may be configured to include a conductive metal material.

The charger 142c may be provided to charge foreign substances in the air. The charger 142c may be provided in an upper portion of the pre-filter 141 to charge foreign substances in the air that have passed through the pre-filter 141. In another expression, the charger 142c may be disposed downstream of the pre-filter 141 in the first passage p1.

The dust collector 142b may be provided to collect foreign substances charged by the charger 142c. Electrodes having different polarities may be connected to the charger 142c and the dust collector 142b, respectively. An electric field may be formed between the charger 142c and the dust collector 142b. Foreign substances in the air charged by the charger 142c may be charged to the same pole as the charger 142c or to a pole opposite to the dust collector 142b. Accordingly, foreign substances in the air charged by the charger 142c may be moved to the dust collector 142b by electric force and collected by the dust collector 142b. In addition, floating bacteria, such as bacteria and viruses, floating in the air may be decomposed by such electric force.

The dust collector 142b may be provided on one side of the electric dust collection filter 142, and the charger 142c may be provided on the other side of the electric dust collection filter 142. The charger 142c and the dust collector 142b may be disposed to face each other. As an example, the charger 142c and the dust collector 142b may be disposed to face each other in the vertical direction. The dust collector 142b may be provided on the upper portion of the electric dust collection filter 142, and the charging part 142c may be provided on the lower portion of the electric dust collection filter 142. The dust collector 142b may be disposed downstream of the charger 142c in the first passage p1. The charger 142c may be disposed between the dust collector 142b and the first inlet 111. The dust collector 142b may be disposed between the charger 142c and the first outlet 112.

The pre-filter 141 may be disposed between the first inlet 111 and the electric dust collection filter 142. In another expression, the electric dust collection filter 142 may be disposed between the first outlet 112 and the pre-filter 141.

In the above-described embodiment of the disclosure, it is described as an example that the dust collection filter 140 includes the electric dust collection filter 142, but is not necessarily limited thereto, and the dust collection filter 140 may further include another dust collection filter or may include another dust collection filter instead. For example, the dust collection filter 140 may include various filters such as a HEPA filter.

The air cleaning device 1 may include a control device 50 controlling the operation of the air cleaning device 1. The control device 50 may include a first printed circuit board assembly 52. The first printed circuit board assembly 52 may be configured to control the operation of the air cleaning device 1. The first printed circuit board assembly 52 may be formed by mounting electronic components controlling the operation of the air cleaning device 1 on a printed circuit board. As an example, the first printed circuit board assembly 52 may be a main board.

The first printed circuit board assembly 52 may be disposed in the cleaning unit 10. As an example, the first printed circuit board assembly 52 may be supported on the base 114 of the cleaning unit 10.

The first printed circuit board assembly 52 may be electrically connected to various components disposed in the cleaning unit 10, such as the power supplier 70 and the first fan 150. The first printed circuit board assembly 52 may be electrically connected to a sensor 90.

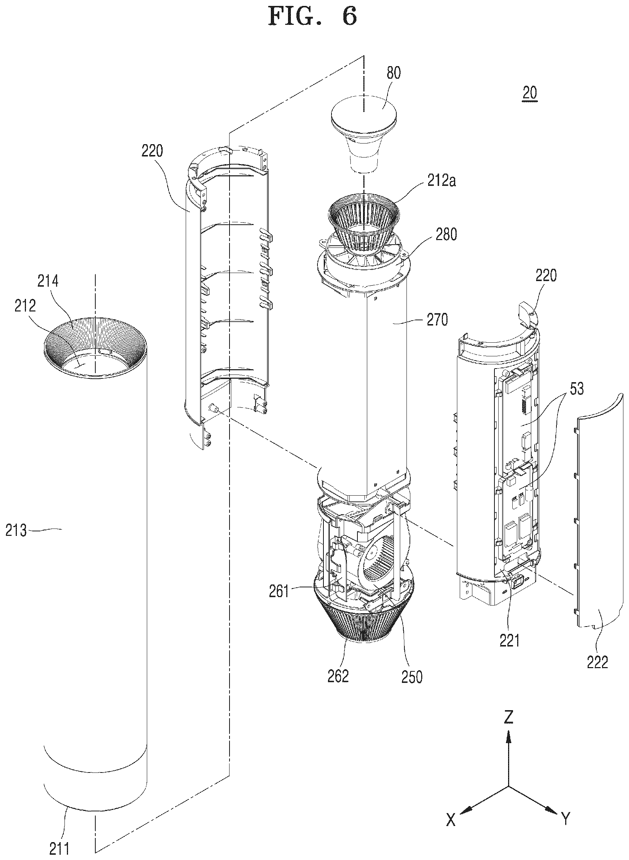

Furthermore, the first printed circuit board assembly 52 may be electrically connected to a second printed circuit board assembly 53 (see FIG. 6, etc.) provided in the sterilizing unit 20, which will be described below. When the first printed circuit board assembly 52 is a main board, the first printed circuit board assembly 52 may be connected to the second printed circuit board assembly 53 by wire, and may transmit a command controlling the operation of the sterilizing unit 20 to the second printed circuit board assembly 53.

However, the first printed circuit board assembly 52 is not limited thereto, and may be disposed at various positions. In addition, the first printed circuit board assembly 52 may be configured in various ways so as to control the operation of the air cleaning device 1.

The air cleaning device 1 may include the power supplier 70 supplying power to various components of the air cleaning device 1. The power supplier 70 may be electrically connected to the first printed circuit board assembly 52. The power supplier 70 may be configured to apply power to various components such as the electric dust collection filter 142.

The power supplier 70 may be disposed in the cleaning unit 10. As an example, the power supplier 70 may be mounted on the first internal frame 120. The first internal frame 120 may include a power supplier mounter 122, and the power supply portion 70 may be mounted in various ways, such as being fastened to the power supplier mounter 122.

The power supplier 70 may receive power from an external power source. The power supplier 70 may be configured to immediately transmit the power supplied from the external power source to components for driving the air cleaning device 1, or accumulate the power supplied from the external power source and then transmit the power to components for driving the air cleaning device 1 when necessary.

However, the power supplier 70 is not limited thereto, and may be disposed at various positions. In addition, the power supplier 70 may be configured in various ways so as to supply power for driving the air cleaning device 1.

The air cleaning device 1 may include the sensor 90. The sensor 90 may be configured to sense a cleaning state of surrounding air, such as an indoor place where the air cleaning device 1 is installed. As an example, the sensor 90 may sense surrounding air through a sensor hole 113a.

The sensor 90 may include various types of sensors. For example, the sensor 90 may include a dust measurement sensor that measures a concentration of dust in the air, a carbon dioxide measurement sensor that measures a concentration of carbon dioxide in the air, a harmful gas measurement sensor that measures a concentration of other harmful gases in the air (e.g., total volatile organic compounds (TVOC), an organic substance measurement sensor that measures a concentration of organic substances in the air, etc.

As an example, the sensor 90 may be disposed in the cleaning unit 10. More specifically, the sensor 90 may be mounted on the first internal frame 120. The first internal frame 120 may include a sensor mounter 121, and the sensor 90 may be mounted in various ways, such as being fastened to the sensor mounter 121.

However, the sensor 90 is not limited thereto, and may be disposed at various positions. In addition, the sensor 90 may be configured in various ways so as to sense the state of the air to be sensed to control the operation of the air cleaning device 1.

As an example, the sensor 90 of the air cleaning device 1 may be provided in the first passage p1. Alternatively, as an example, the sensor 90 of the air cleaning device 1 may be provided in the second passage P2. Alternatively, as an example, the sensor 90 of the air cleaning device 1 may be disposed at a position such as a second internal frame 220 of the sterilizing unit 20, which will be described below.

The configuration of the cleaning unit 10 described above with reference to FIGS. 3 to 5 is only an example for explaining the cleaning unit 10 of the air cleaning device 1 according to the disclosure, and the disclosure is not limited thereto. The cleaning unit 10 of the air cleaning device 1 according to the disclosure may be provided to include various configurations to perform a function of purifying the air flowing along the first passage P1.

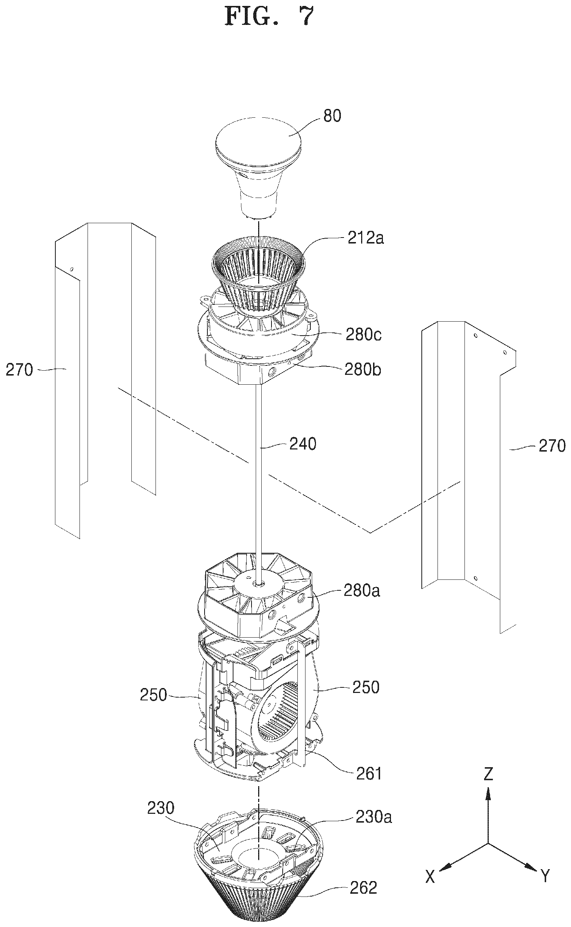

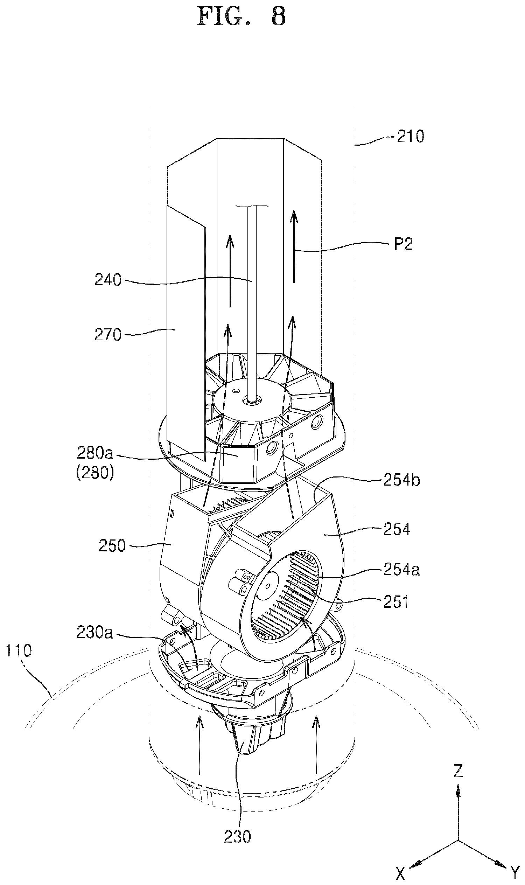

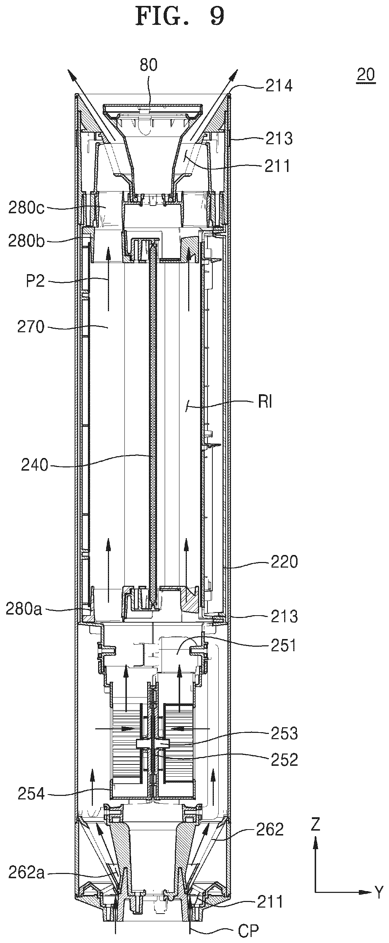

FIG. 6 is an exploded view of some components of the sterilizing unit 20 of the air cleaning device 1 according to an embodiment of the disclosure. FIG. 7 is an exploded view of some components of the sterilizing unit 20 of the air cleaning device 1 according to an embodiment of the disclosure. FIG. 8 is a diagram for explaining air flowing in the sterilizing unit 20 of the air cleaning device 1 according to an embodiment of the disclosure. FIG. 9 is a side cross-sectional view of the sterilizing unit 20 of the air cleaning device 1 according to an embodiment of the disclosure.

Referring to FIGS. 1 and 6 to 9, the sterilizing unit 20 may include the second housing 210, a second fan 250, and an ultraviolet light source 240.

The second housing 210 may form the exterior of the sterilizing unit 20 and may include the second inlet 211 and the second outlet 212. The second housing 210 may be configured to accommodate various components of the sterilizing unit 20 therein.

As an example, the second inlet 211 may be formed in a lower portion of the second housing 210. As an example, the second outlet 212 may be formed in an upper portion of the second housing 210. The second passage P2 extending from the second inlet 211 to the second outlet 212 may be provided inside the second housing 210. As an example, the second passage P2 may be formed to have a shape extending in a vertical direction. Accordingly, air introduced into the second housing 210 through the second inlet 211 may flow upward along the second passage P2.

However, the second inlet 211 and the second outlet 212 are not limited thereto, and may be formed at various positions of the second housing 210. The second passage P2 provided between the second inlet 211 and the second outlet 212 may extend in various directions corresponding to the positions of the second inlet 211 and the second outlet 212.

The second housing 210 may include a second housing body 213 and a second upper cover 214. The second housing body 213 and the second upper cover 214 may each form a part of the exterior of the sterilizing unit 20.

The second housing body 213 may form side surfaces of the sterilizing unit 20 in a horizontal direction. The second housing body 213 may be provided to cover various components of the sterilizing unit 20 in the horizontal direction. The second housing body 213 may be connected to the first upper cover 115. The second housing body 213 may be connected to the intermediate duct 30.

Inside the second housing 210, the second passage P2 may be formed between the second inlet 211 and the second outlet 212. The second housing body 213 may cover the second passage P2 from an outer side of the second passage P2 in a radial direction. In another expression, the second housing body 213 may cover the second passage P2 in a lateral direction thereof in the horizontal direction.

The second housing body 213 may extend in a direction parallel to the second flow passage P2. As an example, the second housing body 213 may extend longitudinally in the vertical direction.

The second housing body 213 may be formed in a substantially hollow cylinder shape. A space formed inside the second housing body 213 may have a substantially cylindrical shape. However, the second housing body 213 is not limited thereto, and may be formed to have various shapes in accordance with the external shape of the sterilizing unit 20.

The second inlet 211 and the second outlet 212 may be formed in the second housing body 213. The second inlet 211 may be provided at one end of the second housing body 213 in a direction toward the intermediate duct 30 (see FIG. 2). The second outlet 212 may be provided at the other end of the second housing body 213 opposite to the second inlet 211.

As an example, the second inlet 211 may be formed at a lower end of the second housing body 213. The second outlet 212 may be formed at an upper end of the second housing body 213. However, the second inlet 211 and the second outlet 212 are not limited thereto, and may be formed at various positions.

The second upper cover 214 may be provided on the upper portion of the second housing 210. The second upper cover 214 may form an upper surface of the sterilizing unit 20. The second upper cover 214 may be formed to cover the space inside the second housing 210 from above. The second upper cover 214 may form at least a part of the upper surface of the second housing 210.

The second upper cover 214 may be provided at the second outlet 212. In another expression, the second upper cover 214 may cover a part of the second outlet 212. The second upper cover 214 may be provided to guide the flow of air discharged through the second outlet 212.

The second upper cover 214 may be formed along the circumference of the second outlet 212. Alternatively, the second upper cover 214 may be formed along the circumference of the second housing body 213.

The second upper cover 214 may have a shape increasing in width in an outer edge direction. For example, the second upper cover 214 may have a shape increasing in width upward. Accordingly, the second upper cover 214 may guide the air discharged through the second outlet 212 to spread outward. In another expression, the second upper cover 214 may be formed to have the shape of approximately a diffuser, but is not limited thereto, and the second upper cover 214 may be configured in various ways so as to form the upper surface of the sterilizing unit 20.

For example, the second housing body 213 and the second upper cover 214 may be formed to be separate from each other. Unlike this, as an example, the second housing body 213 and the second upper cover 214 may be formed integrally with each other.

The sterilizing unit 20 may include a second outlet grill 212a provided at the second outlet 212. The second outlet grill 212a may be formed to have a grill shape so that air may enter and exit through the second outlet 212. For example, the second outlet 212 may be located at an upper side of the second passage P2, and correspondingly, the second outlet grill 212a may be located at the upper side of the second passage P2.

The second outlet grill 212a may be coupled to the second upper cover 214. The second outlet grill 212a may support the second upper cover 214. As an example, the second outlet grill 212a may be coupled to an inner edge of the second upper cover 214. The second outlet grill 212a may be formed along the inner edge of the second upper cover 214.

However, the second outlet grill 212a is not limited thereto, and may be configured in various ways so that air may be discharged through the second outlet 212. Unlike what is described above, the second outlet 212 may be formed on various positions of the second housing 210, such as side surfaces of the second housing body 213. Correspondingly, the second outlet grill 212a may be provided at various positions and may be formed to have various shapes.

The second housing 210 described above is only an example of a configuration that forms the exterior of the sterilizing unit 20 and includes the second passage P2 therein, and the disclosure is not limited thereto.

The sterilizing unit 20 may include the second internal frame 220. The second internal frame 220 may be disposed inside the second housing 210. The second internal frame 220 may be coupled to the inner surface of the second housing 210. The second internal frame 220 may support the inner surface of the second housing 210.

The second internal frame 220 may be provided to support at least some of components of the sterilizing unit 20. For example, the ultraviolet light source 240, a reflective cover 270, a light blocking member 280, etc., may be supported by the second internal frame 220. In addition, the second printed circuit board assembly 53, a control panel 80, etc., may also be supported by the second internal frame 220.

The second internal frame 220 may be covered by the second housing 210. As an example, the second internal frame 220 may be covered in a lateral direction by the second housing body 213 in the horizontal direction. The second inner frame 220 may be covered in an upward direction by the upper cover 214 in the vertical direction.

The second internal frame 220 may be divided into a plurality of frames as shown in FIG. 6, etc. The plurality of frames constituting the second internal frame 220 may form the integrated second internal frame 220 while coupled to each other. Accordingly, the second internal frame 220 may be easily disassembled or assembled, and user accessibility to the internal configuration of the second internal frame 220 may be improved. However, the second internal frame 220 is not limited thereto, and may be integrally formed into a single configuration.

A substantially cylindrical space may be formed inside the second internal frame 220. The ultraviolet light source 240, the reflective cover 270, etc., may be disposed in an internal space of the second internal frame 220. At least a part of the second passage P2 may be provided in the inner space of the second internal frame 220. More specifically, an irradiation region RI, which will be described below, may be provided in the internal space of the second internal frame 220.

However, the second internal frame 220 is not limited thereto, and may be formed to have various shapes. For example, a space formed inside the second internal frame 220 may have a shape other than a cylinder shape. Alternatively, as an example, the second internal frame 220 may be provided to support various components of the sterilizing unit 20 without forming a space therein or covering the outer side of the second flow passage P2.

The second internal frame 220 described above is only an example of a configuration provided to support various components of the sterilizing unit 20, and the disclosure is not limited thereto.

The sterilizing unit 20 may include a second unit coupler 230 so as to be coupled to the cleaning unit 10. The second unit coupler 230 may be coupled to a first unit coupler 130 of the cleaning unit 10 so that the cleaning unit 10 and the sterilizing unit 20 may be coupled to each other.

As an example, the second unit coupler 230 may be disposed in a lower portion of the sterilizing unit 20. As an example, the second unit coupler 230 may be formed to have a shape extending toward the first unit coupler 130 of the cleaning unit 10.

The second unit coupler 230 may include at least one hole 230a through which air flowing along the second passage P2 may pass.

The second unit coupler 230 may be supported on a second fan support frame 261. The second unit coupler 230 may be coupled to a second fan guard 262. However, the sterilizing unit 20 is not limited thereto, and may include various configurations so as to be coupled to the cleaning unit 10.

The second fan 250 may be provided inside the second housing 210. The second fan 250 may generate the second passage P2 from the second inlet 211 to the second outlet 212. The second fan 250 may be disposed between the second inlet 211 and the second outlet 212.

The second fan 250 may generate flow. The second fan 250 may generate suction force as it rotates, and air outside the sterilizing unit 20 may be sucked into the second inlet 211 by the suction force of the second fan 250. The air sucked into the second inlet 211 may flow along the second passage P2 and be discharged through the second outlet 212.

The second fan 250 may be disposed between the ultraviolet light source 240, which will be described below, and the second inlet 211. When the second fan 250 is driven, the air sucked into the second inlet 211 may sequentially pass through the second fan 250 and the ultraviolet light source 240 and be discharged through the second outlet 212.

However, the second fan 250 is not limited thereto, and, as an example, may be disposed between the ultraviolet light source 240 and the second outlet 212. Hereinafter, as shown in FIGS. 6 to 9, it will be described on the assumption that the second fan 250 is disposed between the ultraviolet light source 240 and the second inlet 211.

The second fan 250 may include a second blade 251, a second motor 252 that supplies power to the second blade 251, and a second fan rotation shaft 253 that is connected to the second blade 251 and the second motor 252 and transmits power generated by the motor 252 to the second blade 251.

The second fan 250 may include a second fan case 254 on which the second blade 251, the second motor 252, and the second fan rotation shaft 253 are supported. The second blade 251, the second motor 252, and the second fan rotation shaft 253 may be accommodated in the second fan case 254.

More specifically, the second fan case 254 may support the second motor 252 of the second fan 250. The second motor 252 may be fixed to the second fan case 254. The second blade 251 of the second fan 250 is provided to be rotatable around the second fan rotation shaft 253 with respect to the second motor 252, so that the second blade 251 may be provided to be rotatable with respect to the second fan case 254. In another expression, the second blade 251 may be rotatably supported on the second fan case 254.

The second fan case 254 may include a fan inlet 254a through which air is introduced into the second fan case 254, and a fan discharge duct 254b through which air is discharged to the outside of the second fan case 254. The second passage P2 may be provided to penetrate the fan inlet 254a and the fan discharge duct 254b. As the second fan 250 is driven, air in the second passage P2 may flow through the fan inlet 254a and the fan discharge duct 254b.

As shown in FIGS. 6 to 9, a direction in which air is introduced through the fan inlet 254a and direction in which air is discharged through the fan discharge duct 254b may be different in the second fan 250. More specifically, the direction in which air is introduced through the fan inlet 254a may be parallel to the second fan rotation shaft 253. The direction in which air is discharged through the fan discharge duct 254b may be perpendicular to the second fan rotation shaft 253.

In another expression, the fan discharge duct 254b may extend in a direction perpendicular to the second fan rotation shaft 253. The fan discharge duct 254b may be provided to discharge air in the direction perpendicular to the second fan rotation shaft 253 toward the ultraviolet light source 240. The second fan 250 may be configured as a centrifugal fan type.

A plurality of second fans 250 may be provided. The plurality of second fans 250 may have shapes that correspond to each other.

Each of the plurality of second fans 250 may be provided to discharge air in the direction perpendicular to the second fan rotation shaft 253 toward the ultraviolet light source 240. A direction in which air is discharged from each of the plurality of second fans 250 may be inclined to have a certain angle with respect to a direction in which the second passage P2 extends, for example, the vertical direction. At this time, the direction in which air is discharged from some of the plurality of second fans 250 that is inclined with respect to the vertical direction may be opposite to the direction in which air is discharged from the others of the plurality of second fans 250 that is inclined with respect to the vertical direction. More specifically, in each of the plurality of second fans 250, the fan discharge duct 254b may be positioned to deviate from a central axis of the second passage P2 by a certain distance, where an outer end of the fan discharge duct 254b through which air is discharged may have an inclined shape extending to be close to the central axis of the second passage P2.

However, the shape, arrangement, number, etc., of the second fan 250 are not limited thereto, and the second fan 250 may be provided in various ways. As an example, the second fan 250 may be configured as various types of fans, such as an axial fan. As an example, the second fan 250 may be provided as a single unit.

The second fan 250 described above is only an example of a configuration that generates pressure so that air may flow along the second passage P2 of the sterilizing unit 20, and the disclosure is not limited thereto.

The sterilizing unit 20 may include the second fan support frame 261. The second fan 250 may be supported by the second fan support frame 261.

The second fan support frame 261 may be coupled to the second internal frame 220. For example, the second fan support frame 261 may be coupled to a lower portion of the second internal frame 220.

The second fan support frame 261 may be coupled to the second unit coupler 230. As an example, the second fan support frame 261 may be coupled to an upper portion of the second unit coupler 230.

The sterilizing unit 20 may include the second fan guard 262 disposed upstream of the second fan 250 in the second passage P2. The second fan guard 262 may cover one side of the second fan 250 in a direction of the second inlet 211. The second fan guard 262 may be provided between the second fan 250 and the second inlet 211.

The second fan guard 262 may include a fan guard hole 262a. The fan guard hole 262a may be formed to allow air flowing along the second passage P2 to pass therethrough. Air introduced into the second housing 210 through the second inlet 211 may pass through the fan guard hole 262a and flow toward the second fan 250.

The ultraviolet light source 240 may be provided inside the second housing 210. The ultraviolet light source 240 may be configured to irradiate ultraviolet rays to the second passage P2. Ultraviolet rays irradiated from the ultraviolet light source 240 may have a wavelength in a range where sterilization is possible. Floating bacteria, such as bacteria and viruses, present in the air flowing along the second passage P2 may be destroyed and removed by ultraviolet rays irradiated from the ultraviolet light source 240. The ultraviolet light source 240 may perform a function of sterilizing air flowing along the second passage P2. The ultraviolet light source 240 may be configured to irradiate ultraviolet rays with an amount of light of various intensities. For example, the ultraviolet light source 240 may irradiate ultraviolet rays with various intensities, such as a first amount of light and a second amount of light that is stronger than the first amount of light.

The sterilizing unit 20 may include the reflective cover 270 provided to reflect ultraviolet rays irradiated from the ultraviolet light source 240. The reflective cover 270 may cover an outer side of the ultraviolet light source 240. The reflective cover 270 may be provided in the second passage P2.

An inner surface of the reflective cover 270 facing the ultraviolet light source 240 may include a material with high light reflectivity. As an example, a metal material with high light reflectivity may be plated on the inner surface of the reflective cover 270. Alternatively, as an example, the reflective cover 270 may be integrally configured as a plate of the metal material with high light reflectivity.

The reflective cover 270 may have a curved shape to surround the ultraviolet light source 240. As an example, the reflective cover 270 may have a plurality of flat plate shapes, and the plurality of plate shapes may be provided to form a certain angle with each other and surround the ultraviolet light source 240.

A cross-sectional shape of the reflective cover 270 may have a polygonal structure. For example, the cross-sectional shape of the reflective cover 270 may be designed in consideration of the arrangement of the second printed circuit board assembly 53. For example, the cross-sectional shape of the reflective cover 270 may have an asymmetrical shape in which a plate facing the second printed circuit board assembly 53 is relatively larger than the other plates.

As shown in FIG. 7, a plurality of reflective covers 270 may be provided. The plurality of reflective covers 270 may each cover an outer side of the ultraviolet light source 240. In detail, some of the plurality of reflective covers 270 may cover one side of the ultraviolet light source 240, and the others of the plurality of reflective covers 270 may cover the other side of the ultraviolet light source 240.

However, the number of reflective covers 270 is not limited to the plural, and the singular reflective cover 270 may be provided. In addition, the arrangement and shape of the reflective cover 270 are not limited thereto, and the reflective cover 270 may be provided in various ways to reflect ultraviolet rays irradiated from an outer side of the ultraviolet light source 240 to the ultraviolet light source 240.

The second passage P2 may include the irradiation region RI where ultraviolet rays are irradiated from the ultraviolet light source 240. When the second fan 250 is driven, the air introduced into the second housing 210 through the second inlet 211 may be sterilized as it passes through the irradiation region RI so that floating bacteria such as bacteria and viruses may be removed from the air. The irradiation region RI may be provided inside the reflective cover 270. The irradiation region RI may be surrounded by the reflective cover 270 so that an outer side thereof may be covered. As an example, the reflective cover 270 may cover the irradiation region RI in a lateral direction of the horizontal direction.

With the above configuration, ultraviolet rays irradiated from the ultraviolet light source 240 may be reflected by the reflective cover 270 and may not be incident on an external region of the irradiation region RI, and the sterilization efficiency of the light source 240 may be improved.

The sterilizing unit 20 may further include the light blocking member 280 provided to prevent ultraviolet rays irradiated from the ultraviolet light source 240 from being incident to the outside of the irradiation region RI.

The light blocking member 280 may be provided in the second passage P2. The light blocking member 280 may be formed to allow air flowing along the second passage P2 to pass therethrough. In another expression, the light blocking member 280 may be provided to prevent the ultraviolet rays irradiated from the ultraviolet light source 240 from being emitted to the outside of the irradiation region RI, and at the same time, so that air outside the irradiation region RI may flow into the irradiation region RI through the light blocking member 280, or air inside the irradiation region RI may flow to the outside of the irradiation region RI through the light blocking member 280.

As an example, the light blocking member 280 may include a first light blocking member 280a, a second light blocking member 280b, and a third light blocking member 280c. As an example, the first light blocking member 280a may be provided on one side of the irradiation region RI facing the second inlet 211. The second light blocking member 280b and the third light blocking member 280c may be provided on the other side of the irradiation region RI facing the second outlet 212. As shown in FIGS. 6 to 9, the plurality of light blocking members 280b and 280c may be provided on the other side of the irradiation region RI facing the second outlet 212, thereby more efficiently preventing ultraviolet rays from being emitted to the outside of the air cleaning device 1 through the second outlet 212 and being introduced into a living space of the user.

The first light blocking member 280a and the second light blocking member 280b may be provided to support the ultraviolet light source 240. More specifically, the first light blocking member 280a and the second light blocking member 280b may be provided to support both ends of the ultraviolet light source 240.

The irradiation region RI may be provided between the first light blocking member 280a and the second light blocking member 280b. The first light blocking member 280a and the second light blocking member 280b may be provided at both ends of the irradiation region RI. As an example, the first light blocking member 280a and the second light blocking member 280b may be provided at both ends of the irradiation region RI in the vertical direction, respectively.

With the above configuration, ultraviolet rays irradiated from the ultraviolet light source 240 may be blocked by the light blocking member 280 and may not be incident on the external region of the irradiation region RI, and components of the air cleaning device 1 provided in the external region of the irradiation region RI may be prevented from being damaged by ultraviolet rays. In addition, ultraviolet rays may be prevented from being emitted to the outside of the air cleaning device 1 through the second outlet 212, etc., and introduced into the living space of the user.

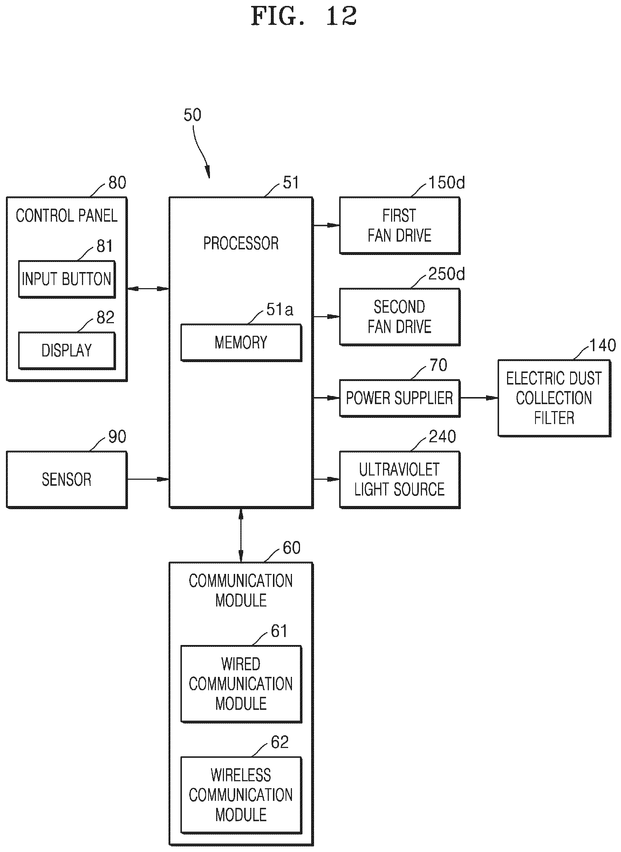

The air cleaning device 1 may include a control panel 80. The control panel 80 may include an input button 81 (see FIG. 12) and a display 82 (see FIG. 12). The control panel 80 may be disposed in the sterilizing unit 20. As an example, the control panel 80 may be supported by the second internal frame 220. As an example, the control panel 80 may be supported by the third light blocking member 280c.

The control panel 80 may be disposed on the upper portion of the sterilizing unit 20. As an example, the sterilizing unit 20 may be provided to cover a part of the second outlet 212. As an example, the second outlet 212 may be covered by the second upper cover 214 in the outer edge direction.

The control device 50 of the air cleaning device 1 may include the second printed circuit board assembly 53. The second printed circuit board assembly 53 may be configured to control the operation of the air cleaning device 1. The second printed circuit board assembly 53 may be formed by mounting electronic components for controlling the operation of the air cleaning device 1 on a printed circuit board.

The second printed circuit board assembly 53 may be disposed in the sterilizing unit 20. As an example, the second printed circuit board assembly 53 may be supported on the second internal frame 220 of the sterilizing unit 20.

The second internal frame 220 may include a board mounter 221 on which the second printed circuit board assembly 53 is mounted. When the second internal frame 220 includes a plurality of frames, the board mounter 221 may be provided on at least some of the plurality of frames constituting the second internal frame 220.

The second internal frame 220 may include a board mounting cover 222 that covers the second printed circuit board assembly 53. The board mounting cover 222 may be detachably coupled to the board mounter 221. The board mounting cover 222 may be provided to protect the second printed circuit board assembly 53.

The second printed circuit board assembly 53 may be electrically connected to various components disposed in the second printed circuit board assembly 53, such as the control panel 80, the ultraviolet light source 240, and the second fan 250. Furthermore, the second printed circuit board assembly 53 may be electrically connected to the first printed circuit board assembly 52 described above. The second printed circuit board assembly 53 may be connected to the first printed circuit board assembly 52 by wire, and may receive a command for controlling the operation of the sterilizing unit 20 from the first printed circuit board assembly 52.

The second printed circuit board assembly 53 may be configured to include a plurality of boards as shown in FIG. 6. In this case, the plurality of boards included in the second printed circuit board assembly 53 may be electrically connected to each other. However, the second printed circuit board assembly 53 is not limited thereto, and may be configured to include a single board.

The characteristics of the second printed circuit board assembly 53 are not limited thereto, and the second printed circuit board assembly 53 may be disposed at various positions. In addition, the second printed circuit board assembly 53 may be configured in various ways so as to control the operation of the air cleaning device 1.

The configuration of the sterilizing unit 20 described above with reference to FIGS. 6 to 9 is only an example for explaining the sterilizing unit 20 of the air cleaning device 1 according to the disclosure, and the disclosure is not limited thereto. The sterilizing unit 20 of the air cleaning device 1 according to the disclosure may be provided to include various configurations to perform the function of purifying the air flowing along the second passage P2.

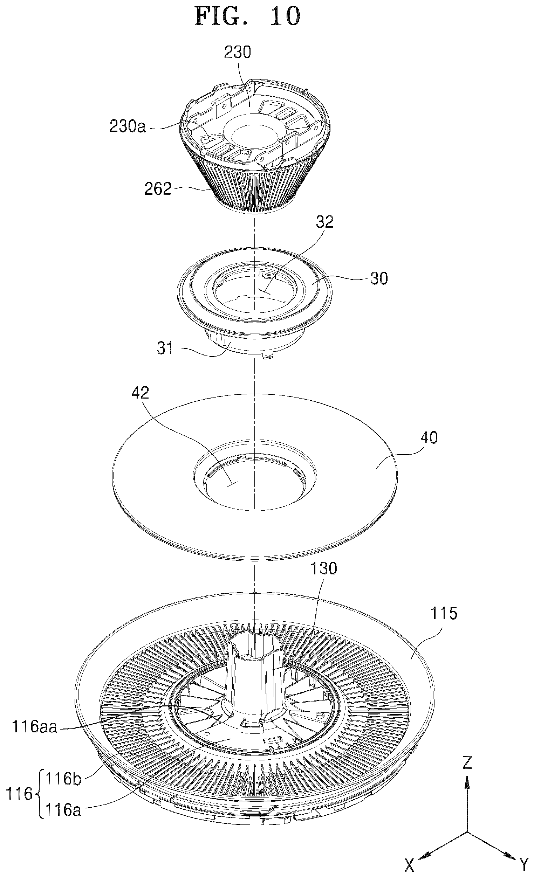

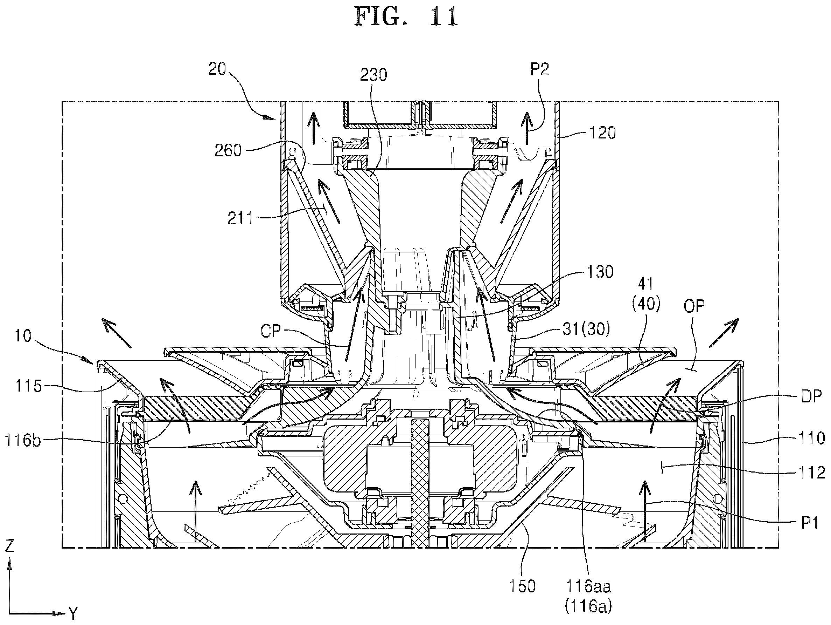

FIG. 10 is a diagram illustrating the intermediate duct 30 of the air cleaning device 1 according to an embodiment of the disclosure and some peripheral components thereof. FIG. 11 is an enlarged cross-sectional view of some components of the air cleaning device 1 according to an embodiment of the disclosure.

Referring to FIGS. 10 and 11, the air cleaning device 1 according to an embodiment of the disclosure may include a connection passage CP connecting the first passage P1 to the second passage P2. The connection passage CP may be connected between the first passage P1 and the second passage P2. The connection passage CP may branch from the first passage P1 and extend toward the second passage P2.

The connection passage CP may be provided to extend from at least a part of the first outlet 112 to the second inlet 211. In another expression, the connection passage CP may be provided between at least a part of the first outlet 112 and the second inlet 211.

At least part of air discharged from the first outlet 112 may be introduced into the connection passage CP. The connection passage CP may be provided so that the air discharged from the first outlet 112 and introduced into the connection passage CP flows into the second passage P2 through the second inlet 211.

The connection passage CP may be provided so that air in the first passage P1 flows into the second passage P2 when the second fan 250 is driven. As an example, when the first fan 150 and the second fan 250 are driven simultaneously, the air introduced into the first passage P1 through the first inlet 111 may be introduced into the second passage P2 by the connection passage CP. As an example, when the first fan 150 is not driven and the second fan 250 is driven, air outside the air cleaning device 1 may be introduced into the first passage P1 through the first outlet 112 by the suction force of the second fan 250, and the air introduced into the first passage P1 may be discharged again through the first outlet 112 and introduced into the connection passage CP. The air introduced into the connection passage CP may be introduced into the second passage P2 through the second inlet 211.

The connection passage CP may extend from at least a part of the first outlet 112 toward the second inlet 211, for example, in the vertical direction as shown in FIG. 10. At least part of air discharged from the first housing 110 through the first outlet 112 may flow upward along the connection passage CP and be introduced into the second housing 210 through the second inlet 211.

The first passage P1, the second passage P2, and the connection passage CP may extend in directions parallel to each other. As an example, the first passage P1, the second passage P2, and the connection passage CP may each extend in the vertical direction of the air cleaning device 1. When the first fan 150 and the second fan 250 are driven simultaneously, at least part of the air introduced into the first housing 110 through the first inlet 111 may flow in approximately parallel directions along the first passage P1, the second passage P2, and the connection passage CP. Accordingly, the efficiency of air flowing within the cleaning unit 10 and the sterilizing unit 20 may be improved.

However, the disclosure is not limited thereto, and a direction in which the connection passage CP extends may vary depending on arrangements of the cleaning unit 10 and the sterilizing unit 20, shapes of the first outlet 112 and the second inlet 211, etc.

The first housing 110 and the second housing 210 may be communicatively connected to each other by the intermediate duct 30. The first passage P1 and the second passage P2 may be communicatively connected to each other through the intermediate duct 30.

The connection passage CP may be formed inside the intermediate duct 30. The intermediate duct 30 may cover an outer side of the connection passage CP. The intermediate duct 30 may cover the connection passage CP in the horizontal direction.

The intermediate duct 30 may guide at least part of the air discharged from the first outlet 112 to be introduced into the second inlet 211. The at least part of the air discharged from the first outlet 112 may be guided to the second inlet 211 by the intermediate duct 30. The intermediate duct 30 may be provided so that air introduced into the intermediate duct 30 flows into the second housing 210 along the connection passage CP.

The intermediate duct 30 may cover at least a part of the first outlet 112. Accordingly, the at least part of the air discharged from the first outlet 112 may be introduced into the intermediate duct 30.

The intermediate duct 30 may cover the entire second inlet 211. Accordingly the air flowing along the intermediate duct 30 may be wholly introduced into the second housing 210.

The intermediate duct 30 may extend in a direction from at least a part of the first outlet 112 toward the second inlet 211, for example, in the vertical direction as shown in FIG. 10. The at least part of the air discharged through the first outlet 112 may be introduced into the intermediate duct 30 and flow upward, and may be introduced into the second housing 210 through the second inlet 211.

Hereinafter, an example of a specific shape of the intermediate duct 30 will be described.

The intermediate duct 30 may include a duct body 31 and a duct hollow portion 32 formed inside the duct body 31. The duct body 31 may have a shape in which an inside thereof is penetrated by the duct hollow portion 32.

The duct hollow portion 32 may extend in a direction from the first housing 110 toward the second housing 210. The duct hollow portion 32 may extend in a direction from the first outlet 112 toward the second inlet 211. As an example, the duct hollow portion 32 may extend in the vertical direction.

The connection passage CP may be formed in the inside of the duct body 31. The duct body 31 may cover the outer side of the connection passage CP. The connection passage CP may be disposed in the duct hollow portion 32.

The duct body 31 may connect the first housing 110 to the second housing 210. The duct body 31 may be connected to each of an upper portion of the first housing 110 and a lower portion of the second housing 210. The duct body 31 may cover at least a part of the first outlet 112. The duct body 31 may cover the entire second inlet 211.

One side of the duct body 31 may be coupled to the lower portion of the second housing 210. One side of the duct body 31 may be coupled to the discharge passage guide 40. As will be described below, the discharge passage guide 40 may be coupled to the upper portion of the first housing 110. Accordingly, the duct body 31 may be directly or indirectly connected to each of the first housing 110 and the second housing 210.

The intermediate duct 30 may be penetrated by the first unit coupler 130. The first unit coupler 130 may penetrate the duct hollow portion 32 and be coupled to the second unit coupler 230. At this time, the connection passage CP may be formed between an inner circumferential surface of the duct body 31 and an outer circumferential surface of the first unit coupler 130.

The configuration of the intermediate duct 30 described above is only an example of the intermediate duct 30 included in the air cleaning device 1 according to the disclosure, and the disclosure is not limited thereto.

In the air discharged from the first outlet 112 other than the part of the air introduced into the connection passage CP, at least another part of the air may be discharged to the outside of the air cleaning device 1. In other words, the at least another part of the air discharged from the first outlet 112 may be discharged to the outside of the cleaning unit 10 and the sterilizing unit 20.

The air cleaning device 1 may include a discharge passage DP that allows at least part of the air discharged from the first outlet 112 to be discharged to the outside of the air cleaning device 1. When the first fan 150 is driven, the at least part of the air discharged from the first outlet 112 may flow along the discharge passage DP.

The discharge passage DP may extend from at least a part of the first outlet 112. The discharge passage DP may branch from the first passage P1 and extend in an outer direction of the air cleaning device 1. A region in which the discharge passage DP branches from the first passage P1 may be located adjacent to the first outlet 112.

The discharge passage DP may be located in an outer edge direction of the connection passage CP. In other words, the discharge passage DP may be disposed to surround the outer side of the connection passage CP. Therefore, part of the air discharged through the first outlet 112 may flow in an inner direction along the connection passage CP, and the other part of the air discharged through the first outlet 112 may flow in an outer direction along the discharge passage DP.

The discharge passage DP may extend in a direction different from the direction in which the connection passage CP extends. As an example, the connection passage CP may extend in the vertical direction, and the discharge passage DP may extend inclined to have a certain angle with respect to the vertical direction.

The discharge passage DP may extend in a direction away from the connection passage CP toward the direction in which air is discharged. In other words, the discharge passage DP may be disposed to discharge air in the direction away from the connection passage CP or the intermediate duct 30. As an example, the discharge passage DP may extend inclined upward toward the outer edge of the first outlet 112.

The air cleaning device 1 may include the discharge passage guide 40 provided to guide the flow of at least part of the air discharged from the first outlet 112. The discharge passage guide 40 may be provided to guide air flowing along the discharge passage DP. The discharge passage guide 40 may guide the flow of air so that part of the air discharged from the first outlet 112 that does not flow into the connection passage CP flows along the discharge passage DP.

One side of the discharge passage DP may be covered by the discharge passage guide 40. The other side of the discharge passage DP may be covered by the first housing 110, for example, the grill assembly or cover 116 of the first outlet 112 or a first upper cover 115.

The discharge passage guide 40 may be disposed to face the first outlet 112 on the outer side of the cleaning unit 10. The discharge passage guide 40 may cover at least a part of the first outlet 112. As an example, the discharge passage guide 40 may cover at least a part of the first outlet 112 from above.

The discharge passage guide 40 may extend in a direction corresponding to the extension direction of the first outlet 112. For example, when the first outlet 112 extends in a horizontal direction of the air cleaning device 1, the discharge passage guide 40 may also extend correspondingly, and accordingly, air flowing along the discharge passage DP may flow in the horizontal direction or a similar direction with respect to the air cleaning device 1 and be discharged to the outside.

The discharge passage guide 40 may be coupled to the cleaning unit 10. The discharge passage guide 40 may be coupled to the first housing 110. As an example, the discharge passage guide 40 may be coupled to the upper portion of the cleaning unit 10 and the upper portion of the first housing 110.

The discharge passage guide 40 may be located in an outer direction of the intermediate duct 30. In other words, the discharge passage guide 40 may be formed to surround the outer side of the intermediate duct 30.

The air cleaning device 1 may include a guide opening OP formed between the outer edge of the discharge passage guide 40 and the outer edge of the first outlet 112. In detail, the guide opening OP may be formed between the outer end of the discharge passage guide 40 and the outer end of the upper cover 115. The guide opening OP may be arranged parallel to the edge of the first outlet 112.

The discharge passage DP may extend from at least a part of the first outlet 112 toward the guide opening OP. The first outlet 112 may be provided in one end of the discharge passage DP, and the guide opening OP may be provided in the other end of the discharge passage DP.

When the first fan 150 and the second fan 250 are driven simultaneously or only the first fan 150 is driven alone, the guide opening OP may function as an outlet through which the air discharged from the first outlet 112 is discharged from the discharge passage DP. When the first fan 150 is not driven and only the second fan 250 is driven independently, the guide opening OP may function as an inlet through which external air is introduced into the discharge passage DP.

By the discharge passage guide 40, the air flowing toward the connection passage CP and the air flowing toward the discharge passage DP may be separated from each other.

Hereinafter, an example of a specific shape of the discharge passage guide 40 will be described.

The discharge passage guide 40 may include a guide surface 41 that guides air flowing along the discharge passage DP. Flow of the at least part of the air discharged through the first outlet 112 may be guided by the guide surface 41.

The guide surface 41 may be formed on one surface of the discharge passage guide 40 facing the discharge passage DP. The guide surface 41 may be formed on one surface of the discharge passage guide 40 facing the first outlet 112.

The guide surface 41 may extend in an inclined direction with respect to the extension direction of the first outlet 112. As an example, the guide surface 41 may extend in an inclined direction with respect to the horizontal direction of the air cleaning device 1. As an example, the guide surface 41 may extend inclined upward toward the outer edge of the discharge passage guide 40. The guide surface 41 may have a shape inclined in a direction corresponding to the first upper cover 115.

The guide surface 41 may be formed to have a concave shape recessed with respect to the discharge passage DP. As an example, the inclination of the guide surface 41 with respect to the horizontal direction may gradually decrease in the outer edge direction of the discharge passage guide 40. As an example, the inclination of the guide surface 41 may have a slope that approaches the horizontal direction toward the direction in which air is discharged along the discharge passage DP. In another expression, the inclination of the guide surface 41 with respect to the horizontal direction may gradually decrease in a direction from the first outlet 112 toward the guide opening OP. The guide surface 41 may be formed to have a curved shape.

The discharge passage guide 40 may include a guide hollow portion 42 formed inside the discharge passage guide 40. The discharge passage guide 40 may have a shape in which an inside thereof is penetrated by the guide hollow portion 42.

The guide hollow portion 42 may extend in a direction from the first housing 110 toward the second housing 210. The guide hollow portion 42 may extend in a direction from the first outlet 112 toward the second inlet 211. As an example, the guide hollow portion 42 may extend in the vertical direction.

The discharge passage guide 40 may be penetrated by the intermediate duct 30. In detail, the guide hollow portion 42 may be penetrated by the intermediate duct 30. The intermediate duct 30 may penetrate the guide hollow portion 42 and connect the first housing 110 to the second housing 210.

As described above, the first unit coupler 130 may penetrate the duct hollow portion 32 of the intermediate duct 30 and be coupled to the second unit coupler 230. Accordingly, the first unit coupler 130 may be disposed to simultaneously penetrate the guide hollow portion 42 and the duct hollow portion 32.

The discharge passage guide 40 may be coupled to the upper portion of the first housing 110, for example, to the cover 116 of the first outlet 112. More specifically, the discharge passage guide 40 may be fastened to a central portion 116a provided on the cover 116 of the first outlet 112 by using a screw (not shown). However, the discharge passage guide 40 is not limited thereto, and may be coupled and fixed to various configurations such as the first housing 110, the second housing 210, and the intermediate duct 30 in various ways.

The configuration of the discharge path guide 40 described above is only an example of a guide of the discharge passage DP included in the air cleaning device 1 according to the disclosure, and the disclosure is not limited thereto.

As described above, the first housing 110 may further include the outlet cover 116 covering the first outlet 112. Air flowing along the first passage p1 may pass through the cover 116 of the first outlet 112 and be discharged from the first housing 110.

Hereinafter, an example of a specific shape of the cover 116 of the first outlet 112 will be described.

The cover 116 of the first outlet 112 may include a central portion 116a and an edge grill 116b provided along an outer edge of the central portion 116a. The central portion 116a may cover an upper center of the first housing 110. The edge grill 116b may cover a circumferential portion of the upper side of the first housing 110. The edge grill 116b may include a penetrating shape. When the first fan 150 is driven, the at least part of the air discharged from the first outlet 112 may pass through the penetrating shape of the edge grill 116b and flow into the discharge passage DP. When the first fan 150 is not driven and the second fan 250 is driven, the air outside the air cleaning device 1 may flow along the discharge passage DP, pass through the penetrating shape of the edge grill 116b, and be introduced into the first housing 110.

One side of the edge grill 116b may be covered by the discharge passage guide 40. As an example, the discharge passage guide 40 may cover at least a part of the edge grill 116b from the upper side. The guide surface 41 of the discharge passage guide 40 may be provided to face at least a part of the edge grill 116b.

The central portion 116a may include a guide portion 116aa extending from the edge grill 116b toward the connection passage CP. The guide portion 116aa may be provided to guide air from the edge grill 116b to the connection passage CP.

The guide portion 116aa may be formed along the circumference of the central portion 116a. The guide portion 116aa may be divided by a plurality of rib structures.

The guide portion 116aa may be formed to have an inclination in a direction parallel to the direction in which the connection passage CP extends toward the connection passage CP. In another expression, an angle formed between an inclination direction of the guide portion 116aa and the extension direction of the connection passage CP may be reduced toward the connection passage CP. As an example, the guide portion 116aa may be formed so that the inclination thereof in the horizontal direction increases toward the connection passage CP. As an example, the guide portion 116aa may be formed to include a concave shape. The guide portion 116aa may be formed to have a curved shape.

The intermediate duct 30 may cover at least a part of the guide portion 116aa. The intermediate duct 30 may be connected to one end of the guide portion 116aa on the connection passage CP.

As an example, the central portion 116a may be formed integrally with the first unit coupler 130 described above. As an example, the first unit coupler 130 may extend upward from the central portion 116a. As an example, the guide portion 116aa may be disposed in a circumferential direction of the first unit coupler 130.

With this configuration, at least part of the air discharged from the first outlet 112 may flow into the connection passage CP along the guide portion 116aa, and at least the other part of the air discharged from the first outlet 112 may flow into the discharge channel DP.

The configuration of the cover 116 of the first outlet 112 described above is only an example of a cover of the first outlet 112 included in the air cleaning device 1 according to the disclosure, and the disclosure is not limited thereto.

In the air cleaning device 1 according to the embodiment of the disclosure described above, at least part of the air from which foreign substances have been removed by the cleaning unit 10 may be transferred to the sterilizing unit 20. The air transferred to the sterilizing unit 20 is sterilized by the ultraviolet light source 240 and is discharged to the outside through the second outlet 212.

FIG. 12 is a block diagram illustrating a configuration of the air cleaning device 1 according to an embodiment of the disclosure.

Referring to FIG. 12, the air cleaning device 1 according to an embodiment of the disclosure may include the control device 50 controlling the configuration of the air cleaning device 1. The control device 50 may include a processor 51. The processor 51 may be configured by the above-described first printed circuit board assembly, second printed circuit board assembly, etc. As an example, the processor 51 may be electrically connected to each of the power supplier 70, a first fan drive 150d, a second fan drive 250d, the electric dust collection filter 142, and the ultraviolet light source 240. As an example, the processor 51 may be configured to control the operation of each of the power supplier 70, the first fan drive 150d, the second fan drive 250d, the electric dust collection filter 142, and the ultraviolet light source 240.

The control panel 80 may provide a user with a user interface for interaction with the user. FIGS. 1 and 7 illustrate a structure in which the control panel 80 is disposed on an upper portion of the sterilizing unit 20, but the control panel 80 is not limited thereto and may be provided at various positions in the air cleaning device 1.

The control panel 80 may include the input button 81 and/or the display 82.

The input button 81 may obtain a user input related to the operation of the air cleaning device 1. For example, the input button 81 may include a power button for turning on/off the operation of the air cleaning device 1, an air volume button for controlling air volume of the air cleaning device 1, a button for selecting an operation mode of the air cleaning device 1, etc.

The display 82 may obtain operation information of the air cleaning device 1 from the processor 51 and display the operation information of the air cleaning device 1.

For example, the display 82 may display the operation mode of the air cleaning device 1 selected by the user. The display 82 may display the air volume of the air cleaning device 1. In addition, the display 82 may display information (e.g., concentrations of dust, gas, organic substances, etc., in the air) about the state of air measured by the sensor 90.

The control panel 80 may include a touch screen in which a display and a touch pad are integrated.

The sensor 90 may sense surrounding air of the air cleaning device 1 and measure a cleaning state of the surrounding air. In another expression, the sensor 90 may measure a cleaning state of an indoor place where the air cleaning device 1 is installed.

The sensor 90 may transmit an electrical signal corresponding to the measured cleaning state to the processor 51. The processor 51 may identify the cleaning state of the surrounding air of the air cleaning device 1 based on the electrical signal received from the sensor 90.

The cleaning state of the surrounding air measured by the sensor 90 or information thereabout may include at least one of a concentration of dust or a concentration of floating bacteria. The cleaning state of the surrounding air may further include a concentration of carbon dioxide in the air, a concentration of other harmful gases (e.g., TVOC), a concentration of organic substances in the air, etc.

For example, the sensor 90 may be configured to measure at least one of the concentration of dust or the concentration of floating bacteria in the surrounding air of the air cleaning device 1.

As an example, a plurality of sensors 90 are provided, and some of the sensors 90 may measure the concentration of dust in the surrounding air, and the other sensors 90 may measure the concentration of floating bacteria of the surrounding air. For example, the sensors 90 may include a single sensor having a function of measuring the concentration of dust in the surrounding air and a function of measuring the concentration of floating bacteria in the surrounding air.

As another example, the sensors 90 may include the sensor 90 that measures the concentration of dust in the surrounding air of the air cleaning device 1 and may not include the sensor 90 that measures the concentration of floating bacteria.

The sensor 90 may be disposed in the cleaning unit 10. The sensor 90 may be disposed in the first internal frame 120 and may sense the surrounding air through a sensing hole provided in the first housing 110. However, a position of the sensor 90 is not limited thereto and the sensor 90 may be disposed at various positions. For example, the sensor 90 may be provided in the sterilizing unit 20 to sense the surrounding air. For example, the sensor 90 may be provided in each of the cleaning unit 10 and the sterilizing unit 20.

The cleaning unit 10 may include the first fan drive 150d electrically connected to the first fan 150. The first fan drive 150d may receive an electrical signal related to driving of the first fan 150 from the processor 51. The first fan drive 150d may control the driving of the first fan 150 based on the electrical signal received from the processor 51.

The sterilizing unit 20 may include the second fan drive 250d electrically connected to the second fan 250. The second fan drive 250d may receive an electrical signal related to driving of the second fan 250 from the processor 51. The second fan drive 250d may control the driving of the second fan 250 based on the electrical signal received from the processor 51.

A communication module 60 may exchange data with external devices such as a server and/or a user device under the control of the processor 51. The communication module 60 may include a wired communication module 61 that exchanges data with external devices by wired, and a wireless communication module 62 that exchanges data with external devices wirelessly.

The wired communication module 61 may connect to a wired communication network and communicate with external devices over the wired communication network. For example, the wired communication module 61 may connect to the wired communication network through Ethernet (IEEE 802.3 technical standard) and receive data from external devices over the wired communication network.

The wireless communication module 62 may communicate wirelessly with a base station or an access point (AP) and may connect to a wired communication network through the base station or the AP. The wireless communication module 62 may also communicate with external devices connected to the wired communication network via the base station or the AP. For example, the wireless communication module 62 may wirelessly communicate with the AP by using WiFi^{™} (IEEE 802.11 technical standard), or may communicate with the base station by using CDMA, WCDMA, GSM, Long Term Evolution (LTE), WiBro, etc. The wireless communication module 62 may also receive data from external devices via the base station or the AP.

Furthermore, the wireless communication module 62 may directly communicate with external devices. For example, the wireless communication module 62 may wirelessly receive data from external devices by using Wi-Fi, Bluetooth^{™} (IEEE 802.15.1 technical standard), ZigBee^{™} (IEEE 802.15.4 technical standard), etc.

As described above, the communication module 60 may exchange data with external devices. The communication module 60 may transmit data received from external devices to the processor 51 and transmit data received from the processor 51 to external devices.

The processor 51 may generate a control signal to control the operation of the air cleaning device 1. The processor 51 may include a memory 51a that stores program and data for generating the control signal. The processor 51 may include one or two or more processors 51, and the memory 51a may be provided integrally with or separately from the processor 51.

The processor 51 may process data and/or signals according to the program stored in the memory 51a and provide the control signal to each component of the air cleaning device 1 based on processing results.

For example, the processor 51 may provide the control signal to each of the cleaning unit 10 and the sterilizing unit 20. For example, the processor 51 may provide a control signal for driving the air cleaning device 1 to each of the first fan drive 150d of the cleaning unit 10, the electric dust collection filter 140, the second fan drive 250d and the ultraviolet light source 240 of and the sterilizing unit 20, etc. The processor 51 controls the cleaning unit 10 and the sterilizing unit 20 simultaneously or individually, and accordingly, the air cleaning device 1 may provide a plurality of operation modes.

The air cleaning device 1 may provide a simultaneous mode 1A, individual modes 1B and 1C, and a standby mode 1D.

When the air cleaning device 1 is in the simultaneous mode 1A, the air cleaning device 1 may operate both the cleaning unit 10 and the sterilizing unit 20. Operation of the cleaning unit 10 may mean an operation to convert air into the cleaning state, and operation of the sterilizing unit 20 may mean an operation to convert air into a sterilizing state.

The operation of the cleaning unit 10 may include a state in which the first fan 150 rotates and a state in which power is applied to the electric dust collection filter 142. In order for the cleaning unit 10 to operate, the processor 51 may control the first fan drive 150d to rotate the first fan 150, and may control the power supplier 70 to apply power to the electric dust collection filter 142.

However, the operation of the cleaning unit 10 is not limited thereto, and may be partially changed in accordance with the configuration or control method of the cleaning unit 10. For example, when the cleaning unit 10 is a dust collection filter capable of collecting dust without applying power, the operation of the cleaning unit 10 may include the state in which the first fan 150 rotates, and may not be in a state in which power is not applied to the dust collection filter.

The operation of the sterilizing unit 20 may include a state in which the second fan 250 rotates and a state in which power is applied to the ultraviolet light source 240. In order for the sterilizing unit 20 to operate, the processor 51 may control the second fan drive 250d to rotate the second fan 250 and control the ultraviolet light source 240 to irradiate ultraviolet rays to an irradiation region. However, the operation of the sterilizing unit 20 is not limited thereto, and may be partially changed in accordance with the configuration or control method of the sterilizing unit 20.

When the air cleaning device 1 is in the individual modes 1B and 1C, the air cleaning device 1 may selectively operate one of the cleaning unit 10 and the sterilizing unit 20. The individual modes 1B and 1C may include the first individual mode 1B in which the cleaning unit 10 operates and the sterilizing unit 20 does not operate, and the second individual mode 1C in which the cleaning unit 10 does not operate and the sterilizing unit 20 operates.

When the air cleaning device 1 is in the first individual mode 1B, in order for the cleaning unit 10 to operate, the processor 51 may control the first fan drive 150d to rotate the first fan 150, and may control the power supplier 70 to apply power to the electric dust collection filter 142.

When the air cleaning device 1 is in the second individual mode 1C, in order for the sterilizing unit 20 to operate, the processor 51 may control the second fan drive 250d to rotate the second fan 250, and may control the ultraviolet light source 240 to irradiate ultraviolet rays to the irradiation region.

When the air cleaning device 1 is in the standby mode 1D, the air cleaning device 1 may operate neither the cleaning unit 10 nor the sterilizing unit 20. When the air cleaning device 1 is in the standby mode 1D, the communication module 60, the control panel 80, the sensor 90, etc., may operate.

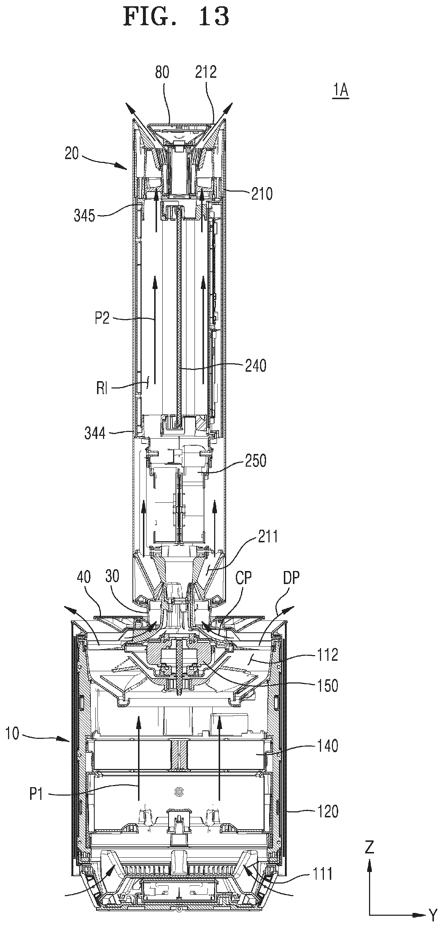

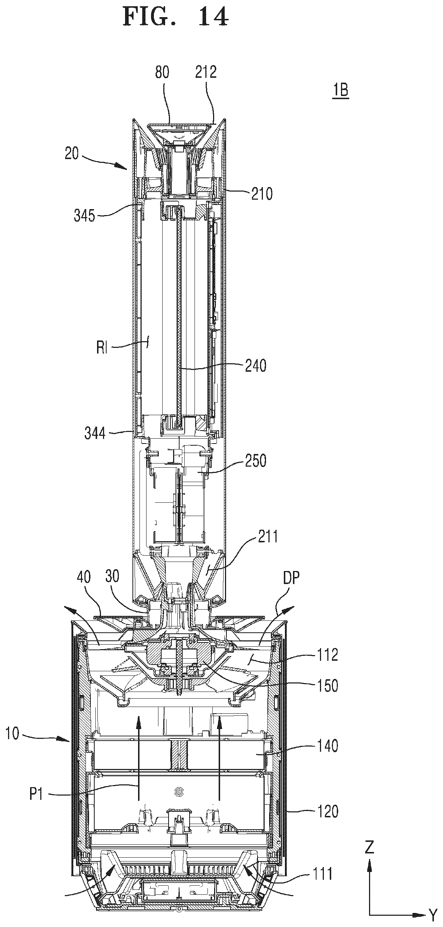

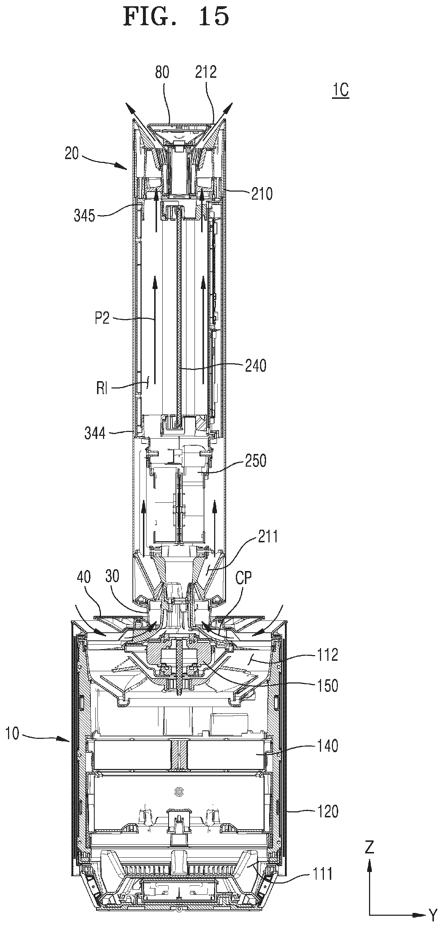

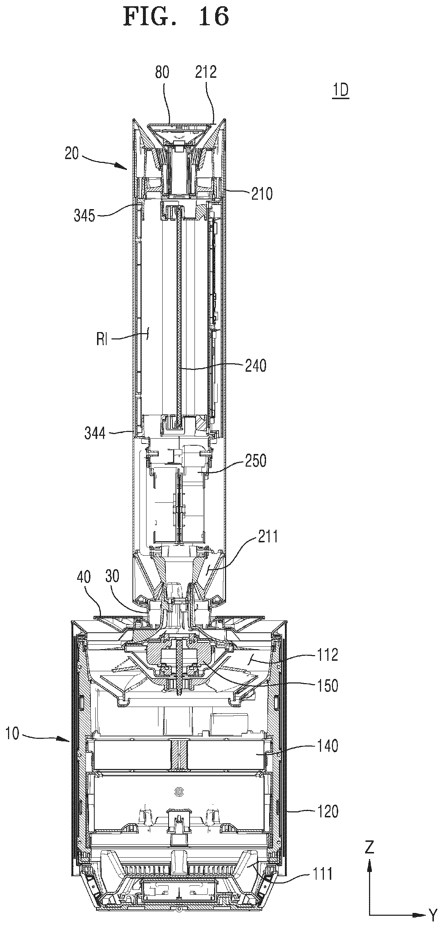

FIG. 13 is a cross-sectional view for explaining an operation of the air cleaning device 1 according to an embodiment of the disclosure in the simultaneous mode 1A. FIG. 14 is a cross-sectional view for explaining an operation of the air cleaning device 1according to an embodiment of the disclosure in the first individual mode 1B. FIG. 15 is a cross-sectional view for explaining an operation of the air cleaning device 1according to an embodiment of the disclosure in the second individual mode 1C. FIG. 16 is a cross-sectional view for explaining an operation of the air cleaning device 1according to an embodiment of the disclosure in the standby mode 1D.

Referring to FIG. 13, the air cleaning device 1 according to an embodiment of the disclosure may operate in the simultaneous mode 1A in which the cleaning unit 10 and the sterilizing unit 20 operate simultaneously. In the simultaneous mode 1A, the cleaning unit 10 may purify air in the first passage p1, and the sterilizing unit 20 may sterilize air in the second passage P2.

When the air cleaning device 1 is in the simultaneous mode 1A, air may be sucked in through the first inlet 111. The air sucked in through the first inlet 111 may move along the first passage p1 and be purified by the dust collection filter 140.

Part of the purified air may pass through the first outlet 112 and be transferred to the second inlet 211, and the remaining purified air may be discharged to the outside through the first outlet 112.

The air transferred to the second inlet 211 may move along the second passage P2 and be sterilized by the ultraviolet light source 240. The sterilized air may be discharged to the outside through the second outlet 212.

When the air cleaning device 1 is in the simultaneous mode 1A, the first fan 150 and the second fan 250 may be simultaneously driven. When the air cleaning device 1 is in the simultaneous mode 1A, the electric dust collection filter 142 and the ultraviolet light source 240 may be simultaneously driven.

The first passage p1 may be provided so that air introduced from the first inlet 111 flows and is discharged to the first outlet 112. As an example, air flowing along the first passage p1 may flow in a vertical direction.

The second passage P2 may be provided so that air sucked from the second inlet 211 flows and is discharged to the second outlet 212. As an example, air flowing along the second passage P2 may flow in the vertical direction.

When the air cleaning device 1 is in the simultaneous mode 1A, at least part of the air discharged from the first outlet 112 may flow and be discharged to the second inlet 211 along the connection passage CP. When the air cleaning device 1 is in the simultaneous mode 1A, at least the other part of the air discharged from the first outlet 112 may be discharged to the outside of the air cleaning device 1 along the discharge passage DP.

When the air cleaning device 1 is in the simultaneous mode 1A, a flow rate of the air purified by the cleaning unit 10 and a flow rate of the air sterilized by the sterilizing unit 20 may be different from each other. In another expression, the flow rate of air flowing along the first passage P1 and the flow rate of air flowing along the second passage P2 may be different from each other. This may vary depending on a flow rate ratio of air flowing along the connection passage CP and the discharge passage DP that branch from the first passage P1.

When the air cleaning device 1 is in the simultaneous mode 1A, air is sucked in through the first inlet 111. The air sucked in through the first inlet 111 moves along the first passage p1 and is purified by the dust collection filter 140. Part of the purified air may pass through the first outlet 112 and be transferred to the second inlet 211, and the remaining purified air may be discharged to the outside through the first outlet 112. The part of the purified air may be transferred to the second inlet 211 along the connection passage CP, and the remaining purified air may be discharged to the outside through the first outlet 112 along the discharge passage DP. The air transferred to the second inlet 211 may move along the second passage P2 and be sterilized by the ultraviolet light source 240, and the sterilized air may be discharged to the outside through the second outlet 212.

In order to improve the sterilization efficiency of the sterilizing unit 20, a flow speed of the air flowing along the second passage P2 may be appropriately set in consideration of an amount and wavelength of ultraviolet rays irradiated from the ultraviolet light source 240. When the flow speed of the air flowing along the second passage P2 is too fast, the air may not be sufficiently sterilized by ultraviolet rays. Conversely, when the flow speed of the air is too slow, a sterilization speed of the sterilizing unit 20 may slow down, which may cause deterioration of the sterilization efficiency.

In order to optimize the flow speed of the air flowing along the second passage P2, the flow rate of the air transferred to the second passage P2 through the connection passage CP may need to be appropriately set. When the air cleaning device 1 is in the simultaneous mode 1A, in order to optimize the flow rate of the air transferred to the second passage P2, a ratio of the flow rate of air flowing along the connection passage CP and the flow rate of air flowing along the discharge passage DP may be appropriately set.

For example, in order to optimize the sterilization efficiency by ultraviolet rays irradiated from the ultraviolet light source 240, the flow rate of the air flowing along the second passage P2 may be set to about 0.5 cubic meter per minute (CMM). In consideration of the flow rate of air and cross-sectional area of the second passage P2, the flow rate of air sucked into the second passage P2 may be set.

The flow rate of air introduced into the connection passage CP from the first passage P1 may be less than the flow rate of air introduced into the discharge passage DP from the first passage P1. For example, the flow rate of air introduced into the connection passage CP from the first passage P1 may be less than 1/4 of the flow rate of air introduced into the discharge passage DP from the first passage P1. For example, the flow rate of air introduced into the connection passage CP from the first passage P1 and the flow rate of air introduced into the discharge passage DP from the first passage P1 may have a ratio of approximately 20 to 80.

The above-described values of the flow rate of air, the ratio of the flow rate, etc., are merely an example described to explain a process of setting a flow rate of air in the second passage P2, a ratio of the flow rate of the connection passage CP and the discharge passage DP, etc., so as to improve the purification efficiency of the air cleaning device 1 according to an embodiment of the disclosure, and the disclosure is not limited thereto.

The flow rate of air, the ratio of the flow rate, etc., in the air cleaning device 1 may be set in various ways according to characteristics such as an amount and wavelength of ultraviolet rays irradiated from the ultraviolet light source 240, the length and cross-sectional area of the second passage P2, shapes of the connection passage CP and the discharge passage DP, and characteristics of each of other components constituting the air cleaning device 1.

As described above, the air cleaning device 1 may enable rapid dust removal and sterilization by simultaneously operating the cleaning unit 10 and the sterilizing unit 20.

Referring to FIG. 14, the air cleaning device 1 according to an embodiment of the disclosure may operate in the first individual mode 1B for operating the cleaning unit 10 among the cleaning unit 10 and the sterilizing unit 20. When the air cleaning device 1 is in the first individual mode 1B, the cleaning unit 10 may purify air moving along the first passage p1. At this time, the sterilizing unit 20 does not operate.

When the air cleaning device 1 is in the first individual mode 1B, the first fan 150 of the cleaning unit 10 may be driven, and the second fan 250 of the sterilizing unit 20 may not be driven. The dust collection filter 140 of the cleaning unit 10 may be driven, and the ultraviolet light source 240 of the sterilizing unit 20 may not be driven.

When the air cleaning device 1 is in the first individual mode 1B, air is sucked in through the first inlet 111 as the first fan 150 is driven. The air sucked in through the first inlet 111 may move along the first passage p1 and be purified by the dust collection filter 140. The purified air may be discharged to the outside of the air cleaning device 1 through the first outlet 112.

At this time, because the second fan 250 is not driven, the air discharged from the first outlet 112 may not flow to the second passage P2 through the connection passage CP. When the second fan 250 is a centrifugal fan and is not driven, air may be more efficiently prevented from flowing to the second passage P2.

With the above configuration, the cleaning unit 10 may selectively operate among the cleaning unit 10 and the sterilizing unit 20, and accordingly, unnecessary operation of the sterilizing unit 20 may be stopped, thereby reducing power consumption. In addition, the lifespan of the ultraviolet light source 240 of the sterilizing unit 20 may extend.

Referring to FIG. 15, the air cleaning device 1 according to an embodiment of the disclosure may operate in the second individual mode 1C in which the sterilizing unit 20 operates among the cleaning unit 10 and the sterilizing unit 20. When the air cleaning device 1 is in the second individual mode 1C, the sterilizing unit 20 may sterilize air moving along the second passage P2. At this time, the cleaning unit 10 does not operate.

When the air cleaning device 1 is in the second individual mode 1C, the first fan 150 of the cleaning unit 10 may not be driven, and the second fan 250 of the sterilizing unit 20 may be driven. The dust collection filter 140 of the cleaning unit 10 may not be driven, and the ultraviolet light source 240 of the sterilizing unit 20 may be driven.

When the air cleaning device 1 is in the second individual mode 1C, air is sucked in through the second inlet 211 as the second fan 250 is driven. The air sucked in through the second inlet 211 may move along the second passage P2 and be sterilized by the ultraviolet light source 240. The air sterilized by the ultraviolet light source 240 may be discharged to the outside of the air cleaning device 1 through the second outlet 212.

When the air cleaning device 1 is in the second individual mode 1C, because the second fan 250 is driven and the first fan 150 is not driven, the first passage P1 may not be formed inside the cleaning unit 10, and external air of the air cleaning device 1 may be introduced into the sterilizing unit 20 through the second outlet 212, the connection passage CP, and the second inlet 211.

However, the flow of air flowing into the sterilizing unit 20 is not limited thereto and may vary. For example, the sterilizing unit 20 may include a separate inlet (not shown) distinguished from the second inlet 211, and air may be introduced into the sterilizing unit 20 through the separate inlet.

With the above configuration, the sterilizing unit 20 may selectively operate among the cleaning unit 10 and the sterilizing unit 20, and accordingly, unnecessary operation of the cleaning unit 10 may be stopped, thereby reducing power consumption, and preventing noise from occurring due to driving of the first fan 150.

Referring to FIG. 16, the air cleaning device 1 according to an embodiment of the disclosure may operate in the standby mode 1D in which neither the cleaning unit 10 nor the sterilizing unit 20 operate.

When the air cleaning device 1 is in the standby mode 1D, the sterilizing unit 20 and the cleaning unit 10 do not operate. When the air cleaning device 1 is in the standby mode 1D, the first fan 150 of the cleaning unit 10 and the second fan 250 of the sterilizing unit 20 may not be driven. The dust collection filter 140 of the cleaning unit 10 and the ultraviolet light source 240 of the sterilizing unit 20 may not be driven.

When the air cleaning device 1 is in the standby mode 1D, the sensor 90 may operate. When the air cleaning device 1 is in the standby mode 1D, the sensor 90, the control panel 80, the communication module 60, etc., may operate. Accordingly, the air cleaning device 1 may measure a cleaning state of the surrounding air through the sensor 90. The air cleaning device 1 may receive a user input through the control panel 80 or the communication module 60.

With the above configuration, the air cleaning device 1 may reduce power consumption and switch to the simultaneous mode 1A, the individual modes 1B and 1C, or the standby mode 1D in accordance with the user input or the cleaning state of the surrounding air.

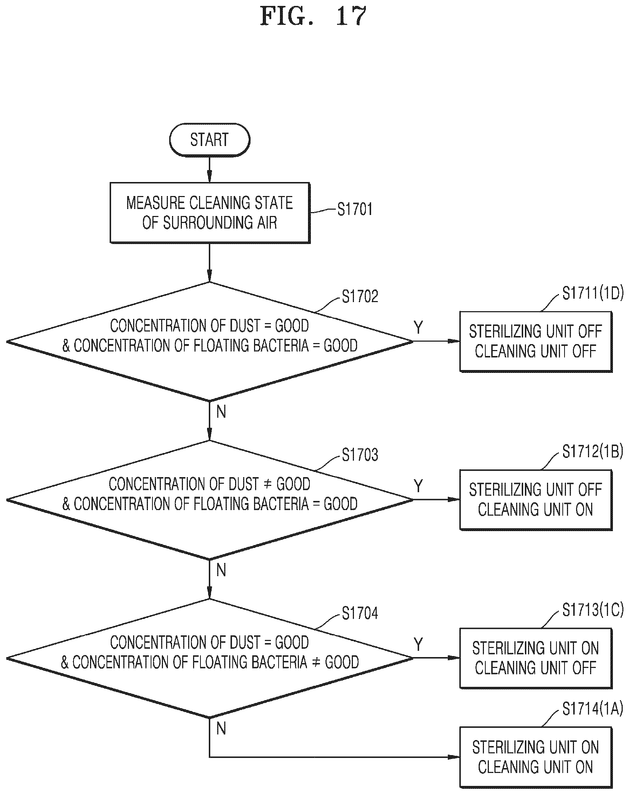

FIG. 17 is a flowchart illustrating a control method of the air cleaning device 1 according to an embodiment of the disclosure.

Referring to FIG. 17, whether the air cleaning device 1 operates the cleaning unit 10 and the sterilizing unit 20 may vary depending on a cleaning state of surrounding air. The cleaning state of the surrounding air may include at least one of a concentration of dust or a concentration of floating bacteria.

The sensor 90 may be configured to measure at least one of the concentration of dust or the concentration of floating bacteria in the surrounding air. For example, the sensor 90 may measure the concentration of dust in the surrounding air and the concentration of floating bacteria in the surrounding air. The sensors 90 may include the sensor 90 that measures the concentration of dust in the surrounding air and the sensor 90 that measures the concentration of floating bacteria in the surrounding air. However, the configuration of the sensor 90 is not limited thereto, and the sensor 90 may be a single sensor 90 having both a function of measuring the concentration of dust in the surrounding air and a function of measuring the concentration of floating bacteria in the surrounding air.

When the air cleaning device 1 starts operating, the sensor 90 may measure a cleaning state of the surrounding air (S1701). For example, the sensor 90 may measure the concentration of dust and the concentration of floating bacteria in the surrounding air. The processor 51 may determine an operation mode of the air cleaning device 1 based on the measured concentration of dust and the measured concentration of floating bacteria. The processor 51 may determine whether to operate the cleaning unit 10 and the sterilizing unit 20 based on the measured concentration of dust and the measured concentration of floating bacteria.

For example, the processor 51 may determine the operation mode of the air cleaning device 1 by determining whether the measured concentration of dust is in a good state and whether the measured concentration of floating bacteria is in a good state. For example, the processor 51 may determine the operation mode of the air cleaning device 1 based on results of comparing the measured concentration of dust with a predetermined first reference concentration and comparing the measured concentration of floating bacteria with a predetermined second reference concentration.

For example, the processor 51 determines whether the measured concentration of dust is in a good state and determines whether to operate the cleaning unit 10. The processor 51 determines whether the measured concentration of floating bacteria is in a good state and determines whether to operate the sterilizing unit 20.

When the measured concentration of dust is in a good state and the measured concentration of floating bacteria is in a good state (S1702), the processor 51 may determine the operation mode of the air cleaning device 1 to be the standby mode 1D (S1711). For example, when the measured concentration of dust is less than the first reference concentration and the measured concentration of floating bacteria is less than the second reference concentration, the processor 51 may determine the operation mode of the air cleaning device 1 to be the standby mode 1D. When the air cleaning device 1 is in the standby mode 1D, the cleaning unit 10 and the sterilizing unit 20 do not operate. The first fan 150 of the cleaning unit 10 does not rotate, and power is not applied to the electric dust collection filter 142. The second fan 250 of the sterilizing unit 20 does not rotate, and power is not applied to the ultraviolet light source 240.

When the measured concentration of dust is not in a good state, but the measured concentration of floating bacteria is in a good state (S1703), the processor 51 may determine the operation mode of the air cleaning device 1 to be the first individual mode 1B (S1712). For example, when the measured concentration of floating bacteria is less than the second reference concentration, but the measured concentration of dust is greater than the first reference concentration, the processor 51 may determine the operation mode of the air cleaning device 1 to be the first individual mode 1B. When the air cleaning device 1 is in the first individual mode 1B, the cleaning unit 10 operates, and the sterilizing unit 20 does not operate. The first fan 150 of the cleaning unit 10 rotates, and power is applied to the electric dust collection filter 142. The second fan 250 of the sterilizing unit 20 does not rotate, and power is not applied to the ultraviolet light source 240.

When the measured concentration of dust is in a good state, but the measured concentration of floating bacteria is not in a good state (S1704), the processor 51 may determine the operation mode of the air cleaning device 1 to be the second individual mode 1C (S1713). For example, when the measured concentration of dust is less than the first reference concentration but the measured concentration of floating bacteria is greater than the second reference concentration, the processor 51 may determine the operation mode of the air cleaning device 1 to be the second individual mode 1C. When the air cleaning device 1 is in the second individual mode 1C, the cleaning unit 10 does not operate, and the sterilizing unit 20 operates. The first fan 150 of the cleaning unit 10 does not rotate, and power is not applied to the electric dust collection filter 142. The second fan 250 of the sterilizing unit 20 rotates, and power is applied to the ultraviolet light source 240.

When the measured concentration of dust is not in a good state and the measured concentration of floating bacteria is not in a good state, the processor 51 may determine the operation mode of the air cleaning device 1 to be the simultaneous mode 1A (S1714). For example, when the measured concentration of dust is greater than the first reference concentration and the measured concentration of floating bacteria is greater than the second reference concentration, the processor 51 may determine the operation mode of the air cleaning device 1 to be the simultaneous mode 1A. When the air cleaning device 1 is in the simultaneous mode 1A, the cleaning unit 10 operates, and the sterilizing unit 20 operates. The first fan 150 of the cleaning unit 10 rotates, and power is applied to the electric dust collection filter 142. The second fan 250 of the sterilizing unit 20 rotates, and power is applied to the ultraviolet light source 240.

In the above-described embodiment of the disclosure, it is described that an example of an operation condition of the cleaning unit 10 includes when the measured concentration of dust is greater than the first reference concentration, and an example of an operation condition of the sterilizing unit 20 includes when the measured concentration of floating bacteria is greater than the second reference concentration, but the operation condition of the cleaning unit 10 and the operation condition of the sterilizing unit 20 are not necessarily limited thereto. The operation condition of the cleaning unit 10 may further include when the measured concentration of dust is equal to the first reference concentration, and the operation condition of the sterilizing unit 20 may further include when the measured concentration of floating bacteria is equal to the second reference concentration.

The air cleaning device 1 may determine whether to operate the cleaning unit 10 and the sterilizing unit 20 in consideration of an operation time of the cleaning unit 10 and an operation time of the sterilizing unit 20, together with the results measured by the sensor 90. Accordingly, the air cleaning device 1 may determine the operation mode thereof in consideration of the operation time of the cleaning unit 10 and the operation time of the sterilizing unit 20.

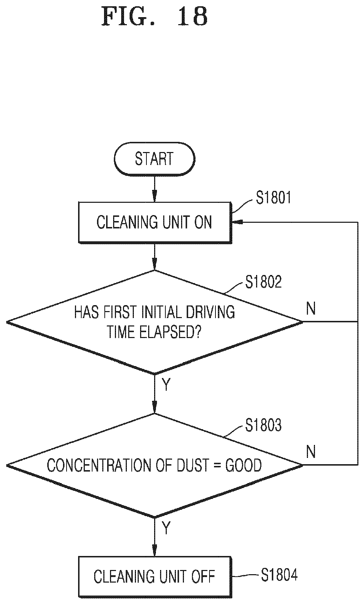

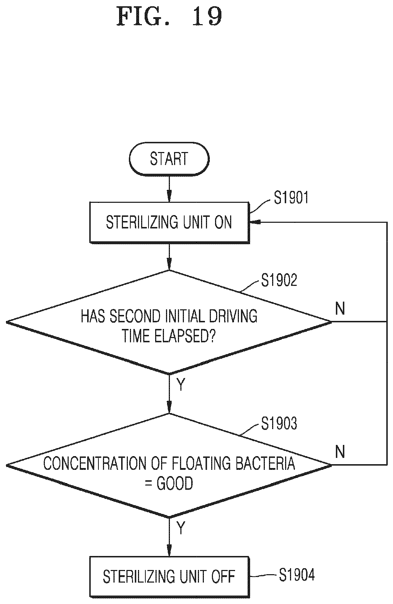

FIG. 18 is a flowchart illustrating an operation of the cleaning unit 10 according to an embodiment of the disclosure. FIG. 19 is a flowchart illustrating an operation of the sterilizing unit 20 according to an embodiment of the disclosure.

Referring to FIGS. 17 and 18, when the air cleaning device 1 is in the simultaneous mode 1A or the first individual mode 1B, the cleaning unit 10 operates (S1801). Here, operating the cleaning unit 10 includes starting to operate the cleaning unit 10 or maintaining the operation of the cleaning unit 10.

The cleaning unit 10 may operate for at least a first predetermined initial driving time. For example, when the cleaning unit 10 starts to operate, the processor 51 determines whether an operation time of the cleaning unit 10 has elapsed the first initial driving time (S1802).

The processor 51 may maintain the cleaning unit 10 in an operation state when the operation time of the cleaning unit 10 has not elapsed the first initial driving time (S1801). When the operation time of the cleaning unit 10 has elapsed the first initial driving time, the processor 51 determines whether a measured concentration of dust is in a good state (S1803). For example, the processor 51 maintains the cleaning unit 10 in the operation state when the measured concentration of dust is greater than a first reference concentration (S1801). The processor 51 stops the cleaning unit 10 when the measured concentration of dust is less than the first reference concentration (S1804).

Referring to FIGS. 17 and 19, when the air cleaning device 1 is in the simultaneous mode 1A or the second individual mode 1C, the sterilizing unit 20 may operate (S1901). Here, operating the sterilizing unit 20 includes starting to operate the sterilizing unit 20 or maintaining the operation of the sterilizing unit 20.

The sterilizing unit 20 may operate for at least a second predetermined initial driving time. For example, when the sterilizing unit 20 starts to operate, the processor 51 determines whether an operation time of the sterilizing unit 20 has elapsed the second initial driving time (S1902).

The processor 51 may maintain the sterilizing unit 20 in an operation state when the operation time of the sterilizing unit 20 has not elapsed the second initial driving time (S1901). When the operation time of the sterilizing unit 20 has elapsed the second initial driving time, the processor 51 determines whether a measured concentration of floating bacteria is in a good state (S1903). For example, the processor 51 maintains the sterilizing unit 20 in the operation state when the measured concentration of floating bacteria is greater than a second reference concentration (S1901). The processor 51 stops the sterilizing unit 20 when the measured concentration of floating bacteria is less than the second reference concentration (S1904).

The second initial driving time of the sterilizing unit 20 may be the same as the first initial driving time of the cleaning unit 10. As another example, the second initial driving time may be different from the first initial driving time. The second initial driving time may be longer than the first initial driving time. For example, the second initial driving time may be 1.5 times or more than the first initial driving time. For example, the second initial driving time may be twice or more than the first initial driving time. For example, the second initial driving time may be 2 to 5 times the first initial driving time.

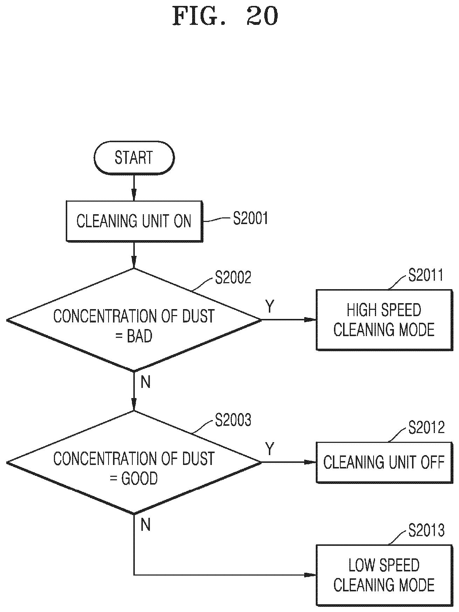

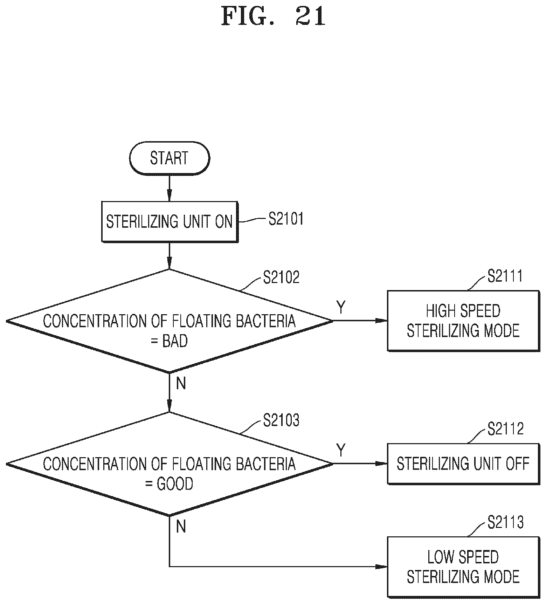

FIG. 20 is a diagram for explaining an operation of the cleaning unit 10 according to a concentration of dust in surrounding air in the air cleaning device 1 according to an embodiment of the disclosure. FIG. 21 is a diagram for explaining an operation of the sterilizing unit 20 according to a concentration of floating bacteria in surrounding air in the air cleaning device 1 according to an embodiment of the disclosure.

Referring to FIGS. 17 and 20, the air cleaning device 1 may operate in the simultaneous mode 1A or the first individual mode 1B (S2001). When the cleaning unit 10 operates, an operation state of the cleaning unit 10 may vary depending on the measured concentration of dust. For example, the processor 51 may determine whether the measured concentration of dust is in a bad state, a normal state, or a good state (S2002 and S2003), and determine the operation state and whether to operate the cleaning unit 10.

For example, the processor 51 may determine that the measured concentration of dust is in a good state when the measured concentration of dust is less than a first reference concentration, may determine that the measured concentration of dust is in a normal state when the measured concentration of dust is greater than the first reference concentration and less than a 1-2 reference concentration, and may determine that the measured concentration of dust is in a bad state when the measured concentration of dust is greater than the 1-2 reference concentration. The 1-2 reference concentration may be greater than the first reference concentration.

For example, the first reference concentration may be 16 *µ*g/m³ or less with respect to a dust (PM 2.5) having a particle size of 2.5 *µ*m or less, and the 1-2 reference concentration may be 36 *µ*g/m³ or more with respect to PM 2.5. The 1-2 reference concentration may be less than or equal to a concentration of dust that is measurable by the sensor 90. However, the first reference concentration and the 1-2 reference concentration are examples, and may be appropriately changed in accordance with settings.

For example, when the processor 51 determines that the measured concentration of dust is in a bad state, the processor 51 may operate the cleaning unit 10 in a high speed cleaning mode (S2011). When the cleaning unit 10 is in the high speed cleaning mode, a rotation speed of the first fan 150 may be the highest. In another expression, the rotation speed of the first fan 150 when the cleaning unit 10 is in the high speed cleaning mode may be higher than the rotation speed of the first fan 150 when the cleaning unit 10 is in a mode other than the high speed cleaning mode. When the cleaning unit 10 is in the high speed cleaning mode, the rotation speed of the first fan 150 may be 75 % or more of the maximum speed at which the first fan 150 is rotatable. When the cleaning unit 10 is in the high speed cleaning mode, the rotation speed of the first fan 150 may be 85 % or more of the maximum speed at which the first fan 150 is rotatable. When the cleaning unit 10 is in the high speed cleaning mode, the rotation speed of the first fan 150 may be 95 % or more of the maximum speed at which the first fan 150 is rotatable.

For example, when the processor 51 determines that the measured concentration of dust is in a normal state, the processor 51 may operate the cleaning unit 10 in a low speed cleaning mode (S2013). The rotation speed of the first fan 150 when the cleaning unit 10 is in the low speed cleaning mode may be lower than the rotation speed of the first fan 150 when the cleaning unit 10 is in the high speed cleaning mode. When the cleaning unit 10 is in the low speed cleaning mode, the rotation speed of the first fan 150 may be less than 75 % of the maximum speed at which the first fan 150 is rotatable. When the cleaning unit 10 is in the low speed cleaning mode, the rotation speed of the first fan 150 may be 50 % or less of the maximum speed at which the first fan 150 is rotatable. When the cleaning unit 10 is in the low speed cleaning mode, the rotation speed of the first fan 150 may be 30 % or less of the maximum speed at which the first fan 150 is rotatable.

For example, when the processor 51 determines that the measured concentration of dust is in a good state, the processor 51 may stop the cleaning unit 10 (S2012). At this time, the first fan 150 may be stopped.

The processor 51 may adjust the intensity of an electrical signal applied to the electric dust collection filter 142 together with the rotation speed of the first fan 150 for efficient cleaning. For example, in order to operate the cleaning unit 10 in the high speed cleaning mode, the processor 51 may increase the rotation speed of the first fan 150 and increase the intensity of the electrical signal applied to the electric dust collection filter 142. For example, in order to operate the cleaning unit 10 in the low speed cleaning mode, the processor 51 may decrease the rotation speed of the first fan 150 and decrease the intensity of the electrical signal applied to the electric dust collection filter 142. Here, the electrical signal may be current or voltage.

Referring to FIGS. 17 and 21, the air cleaning device 1 may operate the sterilizing unit 20 in the simultaneous mode 1A or the second individual mode 1C (S2101). When the sterilizing unit 20 operates, an operation state of the sterilizing unit 20 may vary depending on the measured concentration of floating bacteria. The processor 51 may determine whether the measured concentration of floating bacteria is in a bad state, a normal state, or a good state (S2102 and S2103), and determine the operation state and whether to operate the sterilizing unit 20.

The processor 51 may determine that the measured concentration of floating bacteria is in a good state when the measured concentration of floating bacteria is less than a second reference concentration, may determine that the measured concentration of floating bacteria is in a normal state when the measured concentration of floating bacteria is greater than the second reference concentration and less than a 2-2 reference concentration, and may determine that the measured concentration of floating bacteria is in a bad state when the measured concentration of floating bacteria is greater than the 2-2 reference concentration. The 2-2 reference concentration may be greater than the second reference concentration.

For example, when the measured concentration of floating bacteria is in a bad state, the processor 51 may operate the sterilizing unit 20 in a high speed sterilizing mode (S2111). When the sterilizing unit 20 is in the high speed sterilizing mode, a rotation speed of the second fan 250 may be the highest. In another expression, the rotation speed of the second fan 250 when the sterilizing unit 20 is in the high speed sterilizing mode may be higher than the rotation speed of the second fan 250 when the sterilizing unit 20 is in a mode other than the high speed sterilizing mode. When the sterilizing unit 20 is in the high speed sterilizing mode, the rotation speed of the second fan 250 may be 75 % or more of the maximum speed at which the second fan 250 is rotatable. When the sterilizing unit 20 is in the high speed sterilizing mode, the rotation speed of the second fan 250 may be 85 % or more of the maximum speed at which the second fan 250 is rotatable. When the sterilizing unit 20 is in the high speed sterilizing mode, the rotation speed of the second fan 250 may be 95 % or more of the maximum speed at which the second fan 250 is rotatable.

For example, when the processor 51 determines that the measured concentration of floating bacteria is in a normal state, the processor 51 may operate the sterilizing unit 20 in a low speed sterilizing mode (S2113). The rotation speed of the second fan 250 when the sterilizing unit 20 is in the low speed sterilizing mode may be lower than the rotation speed of the second fan 250 when the sterilizing unit 20 is in the high speed sterilizing mode. When the sterilizing unit 20 is in the low speed sterilizing mode, the rotation speed of the second fan 250 may be less than 75 % of the maximum speed at which the second fan 250 is rotatable. When the sterilizing unit 20 is in the low speed sterilizing mode, the rotation speed of the second fan 250 may be 50 % or less of the maximum speed at which the second fan 250 is rotatable. When the sterilizing unit 20 is in the low speed sterilizing mode, the rotation speed of the second fan 250 may be 30 % or less of the maximum speed at which the second fan 250 is rotatable.

The processor 51 may adjust an intensity of an amount of light irradiated from the ultraviolet light source 240 together with the rotation speed of the second fan 250 for efficient sterilization. For example, in order to operate the sterilizing unit 20 in the high speed sterilizing mode, the processor 51 may increase the rotation speed of the second fan 250 and increase the intensity of the amount of light irradiated from the ultraviolet light source 240. For example, in order to operate the sterilizing unit 20 in the low speed sterilizing mode, the processor 51 may decrease the rotation speed of the second fan 250 and decrease the intensity of the amount of light irradiated from the ultraviolet light source 240.

For example, when the processor 51 determines that the measured concentration of floating bacteria is in a good state, the processor 51 may stop the sterilizing unit 20 (S2112). At this time, the second fan 250 may be stopped.

For example, the second reference concentration may be 500 CFU/m³ or less, and the 2-2 reference concentration may be 800 CFU/m³ or more. The 2-2 reference concentration may be less than or equal to the concentration of floating bacteria that is measurable by the sensor 90. However, the second reference concentration and the 2-2 reference concentration are examples, and may be appropriately changed in accordance with settings.

In the above-described embodiments of the disclosure, an example in which the sensor 90 is capable of measuring both the concentration of dust and the concentration of floating bacteria in the surrounding air, but the air cleaning device 1 according to an embodiment of the disclosure and a control method thereof are not necessarily limited thereto. For example, in the air cleaning device 1 according to an embodiment of the disclosure and the control method thereof, the sensor 90 may measure the concentration of dust in the surrounding air without measuring the concentration of floating bacteria in the surrounding air. For example, the sensor 90 may be a sensor configured to measure the concentration of dust. The sensor 90 may directly measure the concentration of dust and may not directly measure the concentration of floating bacteria.

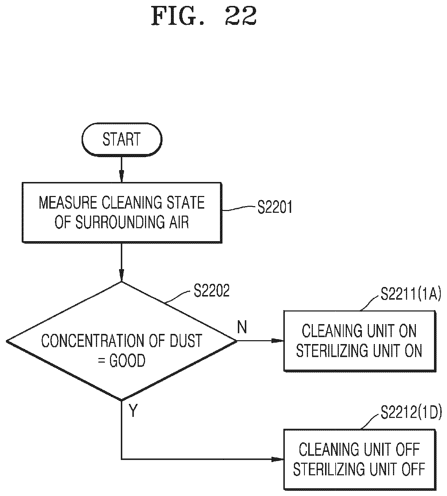

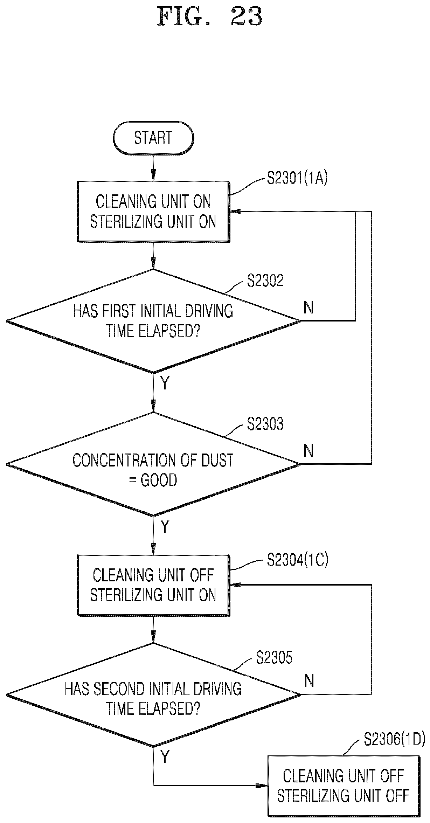

FIG. 22 is a flowchart illustrating a control method of the air cleaning device 1 according to an embodiment of the disclosure when the sensor 90 measures a concentration of dust in surrounding air. FIG. 23 is a flowchart for explaining an operation of the air cleaning device 1 according to an embodiment of the disclosure in the simultaneous mode 1A.

Referring to FIG. 22, in the air cleaning device 1 according to an embodiment of the disclosure, a cleaning state of the surrounding air may be measured by the sensor 90 (S2201). For example, the concentration of dust in the surrounding air may be measured by the sensor 90.

The processor 51 may determine whether the measured concentration of dust is in a good state (S2202). The processor 51 may determine an operation mode of the air cleaning device 1 according to the measured concentration of dust.

For example, when the processor 51 determines that the measured concentration of dust is in a good state, the processor 51 may determine the operation mode of the air cleaning device 1 to be the standby mode 1D (S2212). For example, when the measured concentration of dust is less than a first reference concentration, the processor 51 may determine the operation mode of the air cleaning device 1 to be the standby mode 1D. At this time, the processor 51 may stop the cleaning unit 10 and the sterilizing unit 20.

For example, when the processor 51 determines that the measured concentration of dust is not in a good state, the processor 51 may determine the operation mode of the air cleaning device 1 to be the simultaneous mode 1A (S2211). For example, when the measured concentration of dust is greater than the first reference concentration, the processor 51 may determine the operation mode of the air cleaning device 1 to be the simultaneous mode 1A. At this time, the processor 51 may operate the cleaning unit 10 and the sterilizing unit 20.

Referring to FIG. 23, when the air cleaning device 1 is in the simultaneous mode 1A, the cleaning unit 10 and the sterilizing unit 20 may operate (S2301). Whether to operate the cleaning unit 10 and the sterilizing unit 20 may be determined in consideration of an operation time of at least one of the cleaning unit 10 or the sterilizing unit 20 together with the measured concentration of dust.

For example, the processor 51 determines whether the operation time for which the cleaning unit 10 operates has elapsed a first initial driving time with respect to a time when the air cleaning device 1 starts to operate in the simultaneous mode 1A (S2302).

When the operation time of the cleaning unit 10 has not elapsed the first initial driving time, the processor 51 may maintain the cleaning unit 10 and the sterilizing unit 20 in an operation state (S2301). When the operation time of the cleaning unit 10 has elapsed the first initial driving time, the processor 51 determines whether the measured concentration of dust is in a good state (S2303). For example, the processor 51 determines whether the measured concentration of dust is greater than the first reference concentration.

The processor 51 maintains the cleaning unit 10 and the sterilizing unit 20 in the operation state when the measured concentration of dust is not in a good state (S2301). For example, when the measured concentration of dust is greater than the first reference concentration, the processor 51 maintains the cleaning unit 10 and the sterilizing unit 20 in the operation state. At this time, the air cleaning device 1 may be in the simultaneous mode 1A.

The processor 51 stops the cleaning unit 10 when the measured concentration of dust is in a good state (S2304). The processor 51 stops the cleaning unit 10 when the measured concentration of dust is less than the first reference concentration. At this time, the air cleaning device 1 may be in the first individual mode 1B.

As described above, the processor 51 may compare the measured concentration of dust with the first reference concentration and compare the operation time for which the cleaning unit 10 operates with the first initial driving time to determine whether to operate the cleaning unit 10.

Meanwhile, in a cleaning mechanism in which dust is collected in the cleaning unit 10, dust included in the air moving along the first passage p1 is filtered out while passing through the dust collection filter 140, and thus, dust may be collected relatively quickly. On the other hand, in a sterilizing mechanism in which air is sterilized in the sterilizing unit 20, bacteria included in the air moving along the second passage P2 are sterilized by exposure to ultraviolet rays irradiated from the ultraviolet light source 240, and thus, bacteria may be sterilized relatively slowly. This is because types of bacteria included in the air may vary, bacteria may require different sterilization time, and a degree to which the air passing around the ultraviolet light source 240 is exposed to ultraviolet rays may differ.

In consideration of a difference between the cleaning mechanism of the cleaning unit 10 and the sterilizing mechanism of the sterilizing unit 20, the processor 51 may set the operation time of the sterilizing unit 20 to be longer than the operation time of the cleaning unit 10.

For example, the processor 51 determines whether the operation time for which the sterilizing unit 20 operates has elapsed a second initial driving time with respect to a time when the air cleaning device 1 starts to operate in the simultaneous mode 1A (S2305).

When the operation time of the sterilizing unit 20 has not elapsed the second initial driving time, the processor 51 may stop the cleaning unit 10 and maintain the sterilizing unit 20 in an operation state (S2304). At this time, the air cleaning device 1 may be in the first individual mode 1B.

When the operation time of the sterilizing unit 20 has elapsed the second initial driving time, the processor 51 stops the sterilizing unit 20 (S2306). At this time, the air cleaning device 1 may be in the standby mode 1D.

As described above, the processor 51 may compare the measured concentration of dust with the first reference concentration and compare the operation time for which the sterilizing unit 20 operates with the second initial driving time to determine whether to operate the sterilizing unit 20.

In the embodiment of the disclosure described with reference to FIGS. 22 and 23, an example in which the sensor 90 is configured to directly measure the concentration of dust, and the operation mode of the air cleaning device 1 is determined based on the measured concentration of dust is described. However, although the sensor 90 directly measures the concentration of dust and does not directly measure the concentration of floating bacteria, the air cleaning device 1 may determine the concentration of floating bacteria based on the concentration of dust measured by the sensor 90. For example, when the concentration of dust measured by the sensor 90 or a value in which a correction coefficient is reflected to the concentration of dust satisfies a certain reference range, the air cleaning device 1 may determine the concentration of floating bacteria to be in a good state.

Meanwhile, in the above-described embodiments of the disclosure, an example in which the operation mode of the air cleaning device 1 is automatically selected or determined based on the cleaning state of the surrounding air measured by the sensor 90 is described. However, in the air cleaning device 1 according to an embodiment of the disclosure, the operation mode of the air cleaning device 1 may be selected or determined by a user input.

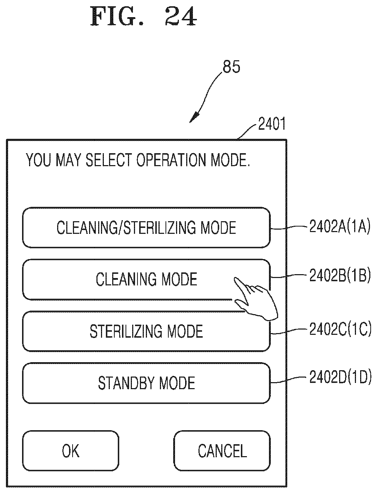

FIG. 24 is a diagram illustrating an operation mode, determined by a user input, of the air cleaning device 1 according to an embodiment of the disclosure.

Referring to FIG. 24, the operation mode of the air cleaning device 1 according to an embodiment of the disclosure may be selected according to the user input. The air cleaning device 1 may be configured to select the simultaneous mode 1A, the first individual mode 1B, the second individual mode 1C, or the standby mode 1D according to the user input.

The user input may be input through an input/output interface 85. The input/output interface 85 may be the control panel 80 of the air cleaning device 1 or the input/output interface 85 of an external device.

The input/output interface 85 may provide a mode setting GUI 2401 through which a user may set the operation mode. The mode setting GUI 2401 may provide menus 2402A, 2402B, 2402C, and 2404D through which the user may set the operation mode.

When the simultaneous mode 1A is selected by the user, the air cleaning device 1 may operate the cleaning unit 10 and the sterilizing unit 20. In this case, the first fan 150 and the dust collection filter 140 of the cleaning unit 10 may operate, and the second fan 250 and the ultraviolet light source 240 of the sterilizing unit 20 may operate.

When the first individual mode 1B is selected by the user, the air cleaning device 1 may operate the cleaning unit 10. In this case, the first fan 150 and dust collection filter 140 of the cleaning unit 10 may operate.

When the second individual mode 1C is selected by the user, the air cleaning device 1 may operate the sterilizing unit 20. In this case, the second fan 250 and the ultraviolet light source 240 of the sterilizing unit 20 may operate.

When the standby mode 1D is selected by the user, the air cleaning device 1 may stop the cleaning unit 10 and the sterilizing unit 20. In another expression, the air cleaning device 1 may not operate the cleaning unit 10 and the sterilizing unit 20.

The user may select an appropriate operation mode of the air cleaning device 1 according to the environment. For example, when dust removal and sterilization are required within a short period of time in a place where many people live or where a lot of sterilization is required, the user may select the simultaneous mode 1A, and select the cleaning unit 10 and the sterilizing unit 20. For example, when dust removal is required but sterilization is not required, the user may select the first individual mode 1B in which only the cleaning unit 10 operates. For example, when sterilization is required but dust removal is not required, the user may select the second individual mode 1C in which only the sterilizing unit 20 operates.

Although reference has been made to embodiments of the disclosure illustrated in the drawings for understanding the disclosure, and specific terms have been used to describe the embodiments thereof, the scope of the disclosure is not limited by the specific terms, and the disclosure will be construed to encompass all embodiments that would normally occur to one of ordinary skill in the art.

Particular implementations described herein merely correspond to embodiments of the disclosure and do not limit the scope of the disclosure in any way. For the sake of brevity of the specification, conventional electronic configurations, control systems, software, and other functional aspects of the systems may be omitted. Furthermore, connecting lines or connectors shown in various figures are intended to represent exemplary functional connections and/or physical or logical couplings between components in the figures, and in an actual device, connections between components may be represented by many alternative or additional functional relationships, physical connections, or logical connections. In addition, an element may not be necessarily essential to the practice of the disclosure unless the element is specifically described as essential," "critical," etc. As used herein, the term such as "comprising", "including" and the like are used to be understood as being an open-ended term for describing embodiments of the disclosure.

The use of the terms "the" and similar referents in the context of describing the disclosure (especially in the context of the following claims) are to be construed to cover both the singular and the plural. Furthermore, recitation of ranges of values herein are merely intended to serve as a shorthand method of referring individually to each separate value falling within the range (unless otherwise indicated herein), and each separate value is incorporated into the specification as when it was individually recited herein. Lastly, operations of methods according to the disclosure described herein may be performed in any suitable order unless clearly specified herein or contradicted by context. The disclosure is not limited to the described order of the operations. The use of any and all examples or exemplary language, e.g., "such as", etc., provided herein is merely intended to describe the disclosure in detail and does not pose a limitation on the scope of the disclosure unless otherwise limited by the claims. Furthermore, various changes and modifications will be readily apparent to one of ordinary skill in the art without departing from the spirit and scope of the disclosure.

The air cleaning device and its control method according to an embodiment of the disclosure may efficiently purify indoor air and reduce power consumption and noise by controlling the cleaning unit and the sterilizing unit to operate simultaneously or individually.

According to an embodiment of the disclosure, an air cleaning device includes a cleaning unit including a first housing including a first inlet and a first outlet, and a first passage extending from the first inlet to the first outlet, a first fan configured to generate an air flow through the first passage, and a dust collection filter in the first passage to collect dust from the air flow generated by the first fan and moving through the first passage, so that the cleaning unit is operable to generate, and collect the dust from, the air flow moving through the first passage; and a sterilizing unit including a second housing including a second inlet connected to the first outlet, a second outlet, and a second passage extending from the second inlet to the second outlet, a second fan configured to generate an air flow through the second passage, and an ultraviolet light source to irradiate ultraviolet rays to the air flow generated by the second fan and moving through the second passage, so that the sterilizing unit is operable to generate, and irradiate the ultraviolet rays to, the air flow moving through the second passage, wherein the air cleaning device is configured to be selectively operable in a simultaneous mode in which both the cleaning unit and the sterilizing unit are operated, first and second individual modes in which one of the cleaning unit and the sterilizing unit is operated alone, and a standby mode in which neither the cleaning unit nor the sterilizing unit are operated, and in the simultaneous mode the first fan and the second fan each generate air flows so that air is sucked in through the first inlet, the air sucked in through the first inlet moves along the first passage and is purified by the dust collection filter, a first portion of the purified air passes through the first outlet and into the second inlet, a second portion of the purified air is discharged to an outside through the first outlet, the first portion of the purified air passed into the second inlet moves along the second passage and is sterilized by the ultraviolet light source, and the sterilized air is discharged to the outside through the second outlet.

According to an embodiment of the disclosure, in the first individual mode, the cleaning unit may be operated and the sterilizing unit may not be operated, and in the second individual mode, the cleaning unit may not be operated and the sterilizing unit may be operated.

According to an embodiment of the disclosure, when the air cleaning device is in the first individual mode, the first fan may generate an air flow so that air may be sucked in through the first inlet, the air sucked in through the first inlet may move along the first passage and may be purified by the dust collection filter, and the purified air may be discharged to the outside through the first outlet. When the air cleaning device is in the second individual mode, the second fan may generate an air flow so that air may be sucked in through the second inlet via the first outlet, the air sucked in through the second inlet may move along the second passage and may be sterilized by the ultraviolet light source, and the sterilized air may be discharged to the outside through the second outlet.

According to an embodiment of the disclosure, the air cleaning device may further include a sensor configured to measure a cleaning state of surrounding air; and a processor configured to control the cleaning unit and the sterilizing unit to operate simultaneously or individually, based on the cleaning state of the surrounding air measured by the sensor.

According to an embodiment of the disclosure, the sensor may be configured to sense at least one of a concentration of dust and a concentration of floating bacteria in the surrounding air.

According to an embodiment of the disclosure, the processor may be configured to compare the concentration of dust sensed by the sensor with a first reference concentration to determine whether to operate the cleaning unit, and compare the concentration of floating bacteria sensed by the sensor with a second reference concentration to determine whether to operate the sterilizing unit.

According to an embodiment of the disclosure, when the sensed concentration of dust is greater than the first reference concentration, and the sensed concentration of floating bacteria is greater than the second reference concentration, the air cleaning device may be controlled to operate in the simultaneous mode. When the sensed concentration of dust is greater than the first reference concentration, and the sensed concentration of floating bacteria is less than the second reference concentration, the air cleaning device may be controlled to operate in the first individual mode. When the sensed concentration of dust is less than the first reference concentration, and the sensed concentration of floating bacteria is greater than the second reference concentration, the air cleaning device may be controlled to operate in the second individual mode. When the sensed concentration of dust is less than the first reference concentration, and the sensed concentration of floating bacteria is less than the second reference concentration, the air cleaning device may be controlled to operate in the standby mode.

According to an embodiment of the disclosure, the sensor may be configured to sense a concentration of dust in the surrounding air, and the processor may be configured to determine whether to operate the cleaning unit and the sterilizing unit, in consideration of an operation time of at least one of the cleaning unit and the sterilizing unit together with the concentration of dust sensed by the sensor.

According to an embodiment of the disclosure, the processor may be configured to compare the sensed concentration of dust with a first reference concentration, and compare the operation time for which the cleaning unit has operated with a first initial driving time to determine whether to operate the cleaning unit.

According to an embodiment of the disclosure, the processor may be configured to compare the sensed concentration of dust with the first reference concentration, and compare the operation time for which the sterilizing unit has operated with a second initial driving time so as to determine whether to operate the sterilizing unit.

According to an embodiment of the disclosure, when the air cleaning device is in the standby mode, the cleaning unit and the sterilizing unit may not operate, and the sensor may operate.

According to an embodiment of the disclosure, a method of controlling an air cleaning device is provided, the air cleaning device having a cleaning unit including a first housing including a first inlet and a first outlet, and a first passage extending from the first inlet to the first outlet, a first fan configured to generate an air flow through the first passage, and a dust collection filter in the first passage to collect dust from the air flow generated by the first fan and moving through the first passage, so that the cleaning unit is operable to generate, and collect the dust from, the air flow moving through the first passage; and a sterilizing unit including a second housing including a second inlet connected to the first outlet, a second outlet, and a second passage extending from the second inlet to the second outlet, a second fan configured to generate an air flow through the second passage, and an ultraviolet light source to irradiate ultraviolet rays to the air flow generated by the second fan and moving through the second passage, so that the sterilizing unit is operable to generate, and irradiate the ultraviolet rays to, the air flow moving through the second passage, the method including measuring at least one of a concentration of dust and a concentration of floating bacteria in surrounding air of the air cleaning device; and based on results of the measuring, controlling the air cleaning device to operate in one operation mode among a simultaneous mode in which both the cleaning unit and the sterilizing unit are operated, first and second individual modes in which one of the cleaning unit and the sterilizing unit is operated alone, and a standby mode in which neither the cleaning unit nor the sterilizing unit are operated, wherein, in the simultaneous mode the first fan and the second fan each generate air flows so that air is sucked in through the first inlet, the air sucked in through the first inlet moves along the first passage and is purified by the dust collection filter, a first portion of the purified air passes through the first outlet and into the second inlet, a second portion of the purified air is discharged to an outside through the first outlet, the first portion of the purified air passed into the second inlet moves along the second passage and is sterilized by the ultraviolet light source, and the sterilized air is discharged to the outside through the second outlet.

According to an embodiment of the disclosure, in the first individual mode the cleaning unit may be operated and the sterilizing unit may not be operated. In the second individual mode the cleaning unit may not be operated and the sterilizing unit may be operated. When the air cleaning device is in the first individual mode, the first fan may generate an air flow so that air may be sucked in through the first inlet, the air sucked in through the first inlet may move along the first passage and may be purified by the dust collection filter, and the purified air may be discharged to the outside through the first outlet. When the air cleaning device is in the second individual mode, the second fan may generate an air flow so that air may be sucked in through the second inlet via the first outlet, the air sucked in through the second inlet may move along the second passage and may be sterilized by the ultraviolet light source, and the sterilized air may be discharged to the outside through the second outlet.

According to an embodiment of the disclosure, the method may further include determining the one operation mode of the air cleaning device by determining whether to operate the cleaning unit by comparing the measured concentration of dust with a first reference concentration, and determining whether to operate the sterilizing unit by comparing the measured concentration of floating bacteria with a second reference concentration.

According to an embodiment of the disclosure, the method may further include determining the one operation mode of the air cleaning device as the simultaneous mode, the first individual mode, the second individual mode, or the standby mode in consideration of an operation time of the cleaning unit and an operation time of the sterilizing unit..

The air cleaning device and a control method thereof according to an embodiment of the disclosure provide a simultaneous mode in which the cleaning unit and the sterilizing unit operate simultaneously, an individual mode in which the cleaning unit and the sterilizing unit selectively operate, and a standby mode in which the cleaning unit and the sterilizing unit do not operate, thereby reducing power consumption and noise while efficiently purifying indoor air.

## Claims

1. An air cleaning device (1), comprising:
a cleaning unit (10) including:
a first housing (110) including a first inlet (111) and a first outlet (112), and a first passage (P1) extending from the first inlet to the first outlet,
a first fan (150) configured to generate an air flow through the first passage, and
a dust collection filter (140) in the first passage to collect dust from the air flow generated by the first fan and moving through the first passage,
so that the cleaning unit is operable to generate, and collect the dust from, the air flow moving through the first passage; and
a sterilizing unit (20) including:
a second housing (210) including a second inlet (211) connected to the first outlet, a second outlet (212), and a second passage (P2) extending from the second inlet to the second outlet,
a second fan (250) configured to generate an air flow through the second passage, and
an ultraviolet light source (240) to irradiate ultraviolet rays to the air flow generated by the second fan and moving through the second passage,
so that the sterilizing unit is operable to generate, and irradiate the ultraviolet rays to, the air flow moving through the second passage,
wherein the air cleaning device is configured to be selectively operable in a simultaneous mode (1A) in which both the cleaning unit and the sterilizing unit are operated, first and second individual modes (1B, 1C) in which one of the cleaning unit and the sterilizing unit is operated alone, and a standby mode (1D) in which neither the cleaning unit nor the sterilizing unit are operated, and
in the simultaneous mode(1A), the first fan and the second fan each generate air flows so that air is sucked in through the first inlet, the air sucked in through the first inlet moves along the first passage and is purified by the dust collection filter, a first portion of the purified air passes through the first outlet and into the second inlet, a second portion of the purified air is discharged to an outside through the first outlet, the first portion of the purified air passed into the second inlet moves along the second passage and is sterilized by the ultraviolet light source, and the sterilized air is discharged to the outside through the second outlet.

2. The air cleaning device of claim 1, wherein
in the first individual mode (1B), the cleaning unit is operated and the sterilizing unit is not operated, and
in the second individual mode (1C), the cleaning unit is not operated and the sterilizing unit is operated.

3. The air cleaning device of claim 2, wherein
when the air cleaning device is in the first individual mode (1B), the first fan generates an air flow so that air is sucked in through the first inlet, the air sucked in through the first inlet moves along the first passage and is purified by the dust collection filter, and the purified air is discharged to the outside through the first outlet, and
when the air cleaning device is in the second individual mode (1C), the second fan generates an air flow so that air is sucked in through the second inlet via the first outlet, the air sucked in through the second inlet moves along the second passage and is sterilized by the ultraviolet light source, and the sterilized air is discharged to the outside through the second outlet.

4. The air cleaning device of claim 2 or 3, further comprising:
a sensor (90) configured to measure a cleaning state of surrounding air; and
a processor (51) configured to control the cleaning unit and the sterilizing unit to operate simultaneously or individually, based on the cleaning state of the surrounding air measured by the sensor.

5. The air cleaning device of claim 4, wherein
the sensor is configured to sense at least one of a concentration of dust and a concentration of floating bacteria in the surrounding air.

6. The air cleaning device of claim 5, wherein
the processor is configured to:
compare the concentration of dust sensed by the sensor with a first reference concentration to determine whether to operate the cleaning unit, and
compare the concentration of floating bacteria sensed by the sensor with a second reference concentration to determine whether to operate the sterilizing unit.

7. The air cleaning device of claim 6, wherein,
when the sensed concentration of dust is greater than the first reference concentration, and the sensed concentration of floating bacteria is greater than the second reference concentration, the air cleaning device is controlled to operate in the simultaneous mode,
when the sensed concentration of dust is greater than the first reference concentration, and the sensed concentration of floating bacteria is less than the second reference concentration, the air cleaning device is controlled to operate in the first individual mode,
when the sensed concentration of dust is less than the first reference concentration, and the sensed concentration of floating bacteria is greater than the second reference concentration, the air cleaning device is controlled to operate in the second individual mode, and
when the sensed concentration of dust is less than the first reference concentration, and the sensed concentration of floating bacteria is less than the second reference concentration, the air cleaning device is controlled to operate in the standby mode.

8. The air cleaning device of claim 4, wherein
the sensor is configured to sense a concentration of dust in the surrounding air, and
the processor is configured to determine whether to operate the cleaning unit and the sterilizing unit, in consideration of an operation time of at least one of the cleaning unit and the sterilizing unit together with the concentration of dust sensed by the sensor.

9. The air cleaning device of claim 8, wherein
the processor is configured to compare the sensed concentration of dust with a first reference concentration, and compare the operation time for which the cleaning unit has operated with a first initial driving time to determine whether to operate the cleaning unit.

10. The air cleaning device of claim 9, wherein
the processor is configured to compare the sensed concentration of dust with the first reference concentration, and compare the operation time for which the sterilizing unit has operated with a second initial driving time so as to determine whether to operate the sterilizing unit.

11. The air cleaning device of claim 4 or 8, wherein
when the air cleaning device is in the standby mode, the cleaning unit and the sterilizing unit do not operate, and the sensor operates.

12. A method of controlling an air cleaning device, the air cleaning device having a cleaning unit including a first housing including a first inlet and a first outlet, and a first passage extending from the first inlet to the first outlet, a first fan configured to generate an air flow through the first passage, and a dust collection filter in the first passage to collect dust from the air flow generated by the first fan and moving through the first passage, so that the cleaning unit is operable to generate, and collect the dust from, the air flow moving through the first passage; and a sterilizing unit including a second housing including a second inlet connected to the first outlet, a second outlet, and a second passage extending from the second inlet to the second outlet, a second fan configured to generate an air flow through the second passage, and an ultraviolet light source to irradiate ultraviolet rays to the air flow generated by the second fan and moving through the second passage, so that the sterilizing unit is operable to generate, and irradiate the ultraviolet rays to, the air flow moving through the second passage, the method comprising:
measuring at least one of a concentration of dust and a concentration of floating bacteria in surrounding air of the air cleaning device; and
based on results of the measuring, controlling the air cleaning device to operate in one operation mode among a simultaneous mode in which both the cleaning unit and the sterilizing unit are operated, first and second individual modes in which one of the cleaning unit and the sterilizing unit is operated alone, and a standby mode in which neither the cleaning unit nor the sterilizing unit are operated,
wherein, in the simultaneous mode the first fan and the second fan each generate air flows so that air is sucked in through the first inlet, the air sucked in through the first inlet moves along the first passage and is purified by the dust collection filter, a first portion of the purified air passes through the first outlet and into the second inlet, a second portion of the purified air is discharged to an outside through the first outlet, the first portion of the purified air passed into the second inlet moves along the second passage and is sterilized by the ultraviolet light source, and the sterilized air is discharged to the outside through the second outlet.

13. The method of claim 12, wherein
in the first individual mode the cleaning unit is operated and the sterilizing unit is not operated,
in the second individual mode the cleaning unit is not operated and the sterilizing unit is operated,
when the air cleaning device is in the first individual mode, the first fan generates an air flow so that air is sucked in through the first inlet, the air sucked in through the first inlet moves along the first passage and is purified by the dust collection filter, and the purified air is discharged to the outside through the first outlet, and
when the air cleaning device is in the second individual mode, the second fan generates an air flow so that air is sucked in through the second inlet via the first outlet, the air sucked in through the second inlet moves along the second passage and is sterilized by the ultraviolet light source, and the sterilized air is discharged to the outside through the second outlet.

14. The method of claim 12 or 13, further comprising
determining the one operation mode of the air cleaning device by:
determining whether to operate the cleaning unit by comparing the measured concentration of dust with a first reference concentration, and
determining whether to operate the sterilizing unit by comparing the measured concentration of floating bacteria with a second reference concentration.

15. The method of any one of claims 12 to 14, further comprising:
determining the one operation mode of the air cleaning device as the simultaneous mode, the first individual mode, the second individual mode, or the standby mode in consideration of an operation time of the cleaning unit and an operation time of the sterilizing unit.
